# EUROPÄISCHE PATENTANMELDUNG

(11) **EP 2 198 953 A1**
(43) Veröffentlichungstag der Anmeldung: **23.06.2010**
(21) Anmeldenummer: 08021550.2
(22) Anmeldetag: 11.12.2008
(51) Int. Cl.: B01J 20/32, C12N 11/08

(54) **Absorbenspartikel basierend auf porösen Trägern und Polyelekrolytschichten**

(71) Anmelder: Süd-Chemie AG, 80333 München (DE); Capsulution NanoScience AG, 12489 Berlin (DE)
(72) Erfinder: Die Erfindernennung liegt noch nicht vor
(74) Vertreter: Rembold, Hansjörg

(57) **Zusammenfassung**

Die Erfindung betrifft ein Trägermaterial, zumindest umfassend:
a.) einen inerten Trägerkern, welcher eine Oberfläche mit einer positiven oder negativen Gesamtladung aufweist;
b.) eine auf dem inerten Trägerkern angeordnete und sich an diesen anschließende erste Schicht aus einem ersten Polyelektrolyt, welcher eine zur Oberflächenladung des inerten Trägerkerns gleichsinnige Gesamtladung aufweist;
c.) eine auf der ersten Schicht aus einem ersten Polyelektrolyt angeordnete zweite Schicht aus einem zweiten Polyelektrolyt, welcher eine zur Gesamtladung des ersten Polyelektrolyts gegensinnige Gesamtladung aufweist.

## Beschreibung

Die Erfindung betrifft ein Trägermaterial, ein Verfahren zur Herstellung eines solchen Trägermaterials sowie die Verwendung des Trägermaterials zur Immobilisierung von Biomolekülen, sowie zur Auftrennung von Gemischen von Biomolekülen.

Enzyme werden in einer Vielzahl technischer Verfahren als Katalysatoren eingesetzt. So werden große Mengen an Enzymen beispielsweise in Waschmitteln verwendet. Ein anderes Gebiet, auf welchem große Mengen an Enzymen eingesetzt werden, ist die Nahrungsmittelindustrie. Solche Enzyme werden in großen Mengen mit biotechnologischen Verfahren hergestellt. Dabei müssen die Enzyme aus großen Volumina eines Reaktions- bzw. Kulturmediums isoliert werden, wobei das Medium neben den gewünschten Enzymen noch eine Vielzahl anderer Verbindungen enthält. Die Abtrennung und Reinigung muss dabei in der Weise erfolgen, dass kein oder nur ein geringer Verlust in der Aktivität des Enzyms eintritt. Enzyme sind jedoch nur ein Beispiel für wertvolle Verbindungen, die aus biologischen Quellen gewonnen werden. Vor allem für pharmazeutische Anwendungen sind eine ganze Reihe von Verbindungen von Bedeutung, wobei diese ebenfalls mit biotechnologischen Methoden hergestellt werden. Beispiele derartiger Verbindungsklassen sind Peptide, Oligonukleotide oder auch Oligo- und Polysaccharide. Auch hier stellt sich das Problem einer Abtrennung dieser Verbindungen aus dem Kulturmedium und einer weiteren Reinigung.

Neben der Isolierung derartiger aus biologischen Quellen oder mittels biotechnologischer Methoden gewonnener Verbindungen stellt sich auch das Problem, diese Verbindungen anschließend in geeigneter Form bereitzustellen, sodass sie als Arzneimittel verwendet oder als Katalysator in einer Reaktion eingesetzt werden können.

Viele Enzyme verlieren beispielsweise relativ rasch ihre Aktivität, wenn sie in reiner Form in einer Reaktion eingesetzt werden. Zur Stabilisierung werden sie daher oft an eine feste Matrix gebunden, wie beispielsweise eine Polymermatrix. Dies bewirkt als weiteren Vorteil, dass sich das Enzym nach beendeter Umsetzung relativ leicht vom Reaktionsmedium abtrennen lässt, beispielsweise durch eine einfache Filtration. Ferner können solche an eine feste Matrix gebundenen Enzyme auch in Form einer Säulenpackung bereitgestellt werden, welche dann vom Reaktionsmedium durchströmt wird. Für andere Anwendungen, beispielsweise als Arzneimittel, kann es gewünscht sein, die aktive Verbindung über einen längeren Zeitraum hinweg kontrolliert freisetzen zu können. Die aktive Verbindung kann daher an einen geeigneten Träger gebunden werden, von welchem sie dann bei der gewünschten Anwendung langsam wieder abgespalten wird.

Es sind eine Vielzahl von Trägermaterialien entwickelt worden, an welchen sich aus biologischen Quellen gewonnene oder mittels biotechnologischer Verfahren hergestellte Verbindungen binden lassen. Wesentlich sind dabei eine hohe Bindungskapazität, eine schnelle Bindungskinetik sowie eine geringe Deaktivierung der adsorbierten Verbindungen.

In der WO 02/083741 wird ein Verfahren zur Immobilisierung von Enzymen auf einem inerten Träger beschrieben. Dazu wird der Träger mit einem kationischen Polymer belegt. Der inerte Träger besteht aus einem Material, welches ausgewählt ist aus der Gruppe von Siliziumdioxid, Aluminiumoxid, Titanoxid, Diatomeenerde, Kaolin, Glas, organischen Polymeren sowie Zellulose, wobei Siliziumdioxid als Trägermaterial bevorzugt ist. Als kationisches Polymer wird bevorzugt ein Polyamin verwendet. Auf dem Träger wird ein Enzym immobilisiert, wobei bevorzugt Glukoseisomerase eingesetzt wird. Zur Herstellung wird der Träger zunächst mit dem kationischen Polymer umgesetzt, sodass sich dieses auf dem Träger abscheidet. Anschließend wird der Träger mit dem Enzym umgesetzt, wobei die Bedingungen so gewählt werden, dass sich das Enzym an das kationische Polymer anlagert, ohne dass dazu ein Vernetzungsmittel verwendet werden muss.

Nachteilig an diesem Verfahren ist, dass die adsorbierte Menge an kationischem Polymer bei vielen Trägermaterialien sehr gering ist. Die Beladung hängt von der Stärke der negativen Oberflächenladung (ζ-Potential) des Trägermaterials ab. Die Menge des adsorbierten Polykations bestimmt jedoch die Aufnahmekapazität des Trägers für z.B. Enzyme. Die Menge des adsorbierten kationischen Polymers sollte also möglichst groß sein. Des Weiteren hängt die Stärke der Bindung zwischen kationischem Polymer und Träger von der Oberflächenladung des Trägers und der Ladung des kationischen Polymers ab. Werden beispielsweise Silikate als Träger verwendet, so nimmt die Ladung mit sinkendem pH-Wert stark ab, um bei einem pH im Bereich von 2 bis 3 auf null abzusinken. In der Folge verringert sich die Haftung des kationischen Polymers auf der Oberfläche des Trägers. Dadurch kann es zu einer Desorption des Polymers von der Oberfläche des Trägers kommen. Dies kann bei einer Anwendung in lebensmitteltechnologischen Prozessen nicht toleriert werden. Auch bei anderen Anwendungen stört eine solche Ablösung der Polymerschicht vom Träger, da sie eine Verminderung der Enzymaktivität bewirkt. Ähnliche Schwierigkeiten können auch bei hohen Ionenstärken auftreten, da die Ionen des Salzes dann mit dem kationischen Polymer um die Bindungsstellen am Träger konkurrieren.

In der WO 2005/089727 wird ein Verfahren zur Herstellung von CS-Partikeln ("CS" = "core-shell"; Kern-Schale) sowie von befüllten Mikrokapseln beschrieben. Dazu wird zunächst ein poröses Templat, beispielsweise ein poröses Siliziumdioxidpartikel, mit dem zu verkapselnden Wirkstoff beladen. Dies erfolgt beispielsweise, indem das poröse Templat in einer Lösung des Wirkstoffs suspendiert wird. Auf das mit dem Wirkstoff beladene Templat kann dann eine Grundierungsschicht aufgebracht werden. Diese dient dazu, Unregelmäßigkeiten auf der Templatoberfläche, wie sie beispielsweise durch Poren verursacht werden, auszugleichen. Die Grundierung wird so ausgewählt, dass sie nicht in die Poren des Templats eindringt, diese aber für nachfolgende Beschichtungen abdichtet. Auf dem, ggf. mit einer Grundierungsschicht versehenen, porösen Templat wird dann eine Kapselhülle aufgebracht. Dazu werden mehrere Schichten von alternierend geladenen Polyelektrolyten aufgetragen. Die Polyelektrolyten werden bevorzugt jeweils als Einzelschicht aufgetragen. Ggf. kann die Kapselhülle noch vernetzt werden, um die Stabilität der Kapselhülle zu erhöhen. Anschließend wird das poröse Templat aus dem von der Kapselhülle gebildeten Hohlraum herausgelöst. Der auf dem Templat adsorbierte Wirkstoff bleibt zurück, sodass er nun in verkapselter Form vorliegt.

Obwohl bereits eine Vielzahl von Trägern für die Immobilisierung von Biomolekülen entwickelt worden sind, besteht dennoch weiterhin ein großer Bedarf nach derartigen Trägern, sodass für ein vorgegebenes Problem der Immobilisierung eines bestimmten Wirkstoffs ein nach Möglichkeit optimaler Träger zur Verfügung steht. Insbesondere soll der Träger eine stabile Immobilisierung von Biomolekülen, insbesondere Enzymen, auch bei unterschiedlichen Bedingungen ermöglichen. So soll die Immobilisierung über einen breiten pH-Bereich und auch bei hohen Salzkonzentrationen stabil sein.

Der Erfindung lag daher die Aufgabe zugrunde, ein Trägermaterial für die Immobilisierung von Biomolekülen zur Verfügung zu stellen, das vorteilhafte Bindungseigenschaften sowie eine hohe Beständigkeit gegenüber niedrigem pH und hohen Ionenstärken aufweist und eine möglichst breite Anwendbarkeit besitzt.

Diese Aufgabe wird mit einem Trägermaterial mit den Merkmalen des Patentanspruchs 1 gelöst. Vorteilhafte Ausführungsformen des Trägermaterials sind Gegenstand der abhängigen Patentansprüche.

Überraschend wurde gefunden, dass sich auf einem inerten Trägerkern, welcher eine positive oder negative Gesamtladung aufweist, eine Schicht eines gleichsinnig geladenen Polyelektrolyts aufbringen lässt. Damit kann die Anzahl an geladenen Gruppen, die für eine Koordination weiterer Verbindungen, die eine zum Polyelektrolyt bzw. inerten Trägerkern gegensinnige Ladung aufweisen, zur Verfügung stehen, erhöht werden. Erfindungsgemäß wurde also gefunden, dass sich auf einem inerten Träger, der an seiner Oberfläche eine positive Gesamtladung aufweist, ein positiv geladener Polyelektrolyt aufbringen lässt, während sich auf einem inerten Träger, dessen Oberfläche eine negative Gesamtladung aufweist, auch ein negativ geladener Polyelektrolyt aufbringen lässt.

An sich wäre zu erwarten, dass sich gleichsinnige Ladungen abstoßen und sich daher ein gleichsinnig geladener Polyelektrolyt nicht auf der Oberfläche des inerten Trägers adsorbieren lässt und stattdessen separat vom inerten Träger in Lösung verbleibt. Ohne an diese Theorie gebunden sein zu wollen, nehmen die Erfinder an, dass auf der Oberfläche keine gleichmäßige Ladungsverteilung vorliegt, sondern die Ladungsdichte örtliche Schwankungen zeigt oder dass zusätzliche Wechselwirkungen, wie Wasserstoffbrückenbindungen, van-der-Waals- oder auch hydrophobe Wechselwirkungen eine Adsorption gegen die abstoßende Wechselwirkung zwischen dem Polyelektrolyt und der gleichsinnig geladenen Oberfläche des inerten Trägers bewirken können. Es ist also denkbar, dass sich an lokalen Stellen der Oberfläche des inerten Trägers Bindungen zu Gruppen des ersten Polyelektrolyts ausbilden, sodass dieser an die Oberfläche des inerten Trägers koordiniert wird. Unterstützend sind auch entropische Effekte denkbar, da der Polyelektrolyt Lösungsmittelmoleküle von der Oberfläche des Trägers verdrängen kann. Der Grund für die unerwartete Koordination des Polyelektrolyts an die Oberfläche eines gleichsinnig geladenen inerten Trägers ist aber bisher noch nicht bis ins Letzte verstanden. Indem auf die Oberfläche des inerten Trägerkerns zunächst eine Schicht eines gleichsinnig geladenen Polyelektrolyts aufgebracht wird, erhöht sich die Ladung des Trägerkerns, d.h. es stehen mehr Bindungsstellen für gegensinnig geladene Gruppen zur Verfügung.

Auf der Schicht des ersten Polyelektrolyts ist eine Schicht eines zweiten Polyelektrolyts aufgebracht, wobei erster und zweiter Polyelektrolyt ein entgegengesetztes Vorzeichen der Gesamtladung aufweisen. Die Gesamtladung von erstem und zweitem Polyelektrolyt wird wesentlich von geladenen Gruppen bestimmt, die am Polyelektrolyt gebunden sind. So weist ein Polyelektrolyt mit einer positiven Gesamtladung bevorzugt kationische Gruppen auf, während ein Polyelektrolyt mit einer negativen Gesamtladung entsprechend bevorzugt anionische Gruppen aufweist. Es ist darüber hinaus auch möglich, dass der Polyelektrolyt Gruppen mit einem hohen Dipolmoment bzw. einer hohen Polarisierung aufweist, die beispielsweise über Wasserstoffbrückenbindungen eine Koordination des ersten Polyelektrolyts an die Oberfläche des inerten Trägers bewirken.

Durch die Bereitstellung einer hohen Anzahl potentieller Bindungsstellen kann eine im Vergleich zu Trägermaterialien aus dem Stand der Technik höhere Beladung mit dem zweiten Polyelektrolyt erreicht werden. Durch die größere Menge an zweitem Polyelektrolyt, die auf dem inerten Trägerkern aufgebracht werden kann, steht auch eine höhere Anzahl von geladenen oder stark polaren Gruppen zur Verfügung, die wiederum für die Adsorption weiterer Moleküle, beispielsweise von Biomolekülen, wie Peptiden oder Enzymen, zur Verfügung stehen.

Als weiterer Vorteil ist die auf dem inerten Trägerkern aufgebrachte sandwichartige Hülle aus entgegengesetzt geladenen Polyelektrolyten auch stabiler gegenüber Änderungen des pH-Wertes eines Reaktionsmediums oder gegenüber hohen Salzkonzentrationen als eine Hülle, die erzeugt wurde, indem der zweite Polyelektrolyt direkt auf die Oberfläche des inerten Trägerkerns aufgebracht wird, wobei Polyelektrolyt und Oberfläche des Trägerkerns entgegengesetzt geladen sind. Ohne an diese Theorie gebunden sein zu wollen nehmen die Erfinder an, dass die Gründe für die bessere Stabilität des erfindungsgemäßen Trägermaterials einerseits in der höheren Ladung liegen, welche durch das Aufbringen eines gleichsinnig geladenen Polyelektrolyts bereit gestellt wird, und andererseits in der Flexibilität der Polyelektrolytketten, durch welche sich der zweite Polyelektrolyt an die Ladungsverteilung auf der Oberfläche des mit dem ersten Polyelektrolyt beschichteten Trägerkerns anpassen kann, sodass die Wechselwirkung zwischen den geladenen Gruppen optimiert werden kann.

Ein erfindungsgemäßes Trägermaterial mit zumindest zwei Schichten aus Polyelektrolyten mit entgegengesetztem Vorzeichen der Gesamtladung auf einem inerten Trägerkern zeigt eine überraschend hohe Bindungskapazität für Biomoleküle. Ohne an diese Theorie gebunden sein zu wollen, erklären sich die Erfinder diesen Effekt mit einer sehr effizienten Belegung der Oberfläche des inerten Trägerkerns durch die aus Polyelektrolyten gebildete Schicht. Dies kann einerseits, wie erläutert, auf die Bereitstellung einer relativ hohen Anzahl geladener Gruppen und damit von Bindungsstellen auf der Trägeroberfläche zurückgeführt werden. Andererseits wird angenommen, dass sich die aus den Polyelektrolyten gebildete Doppelschicht nicht nur eng an die Oberfläche des inerten Trägers anlegt, sondern auch von der Oberfläche des Trägerkerns weg nach außen ragende Enden der Polyelektrolytketten sowie vom Polyelektrolyt gebildete Schlaufen zu einer Erhöhung der Nanoporosität des Trägermaterials führen, wodurch die Kapazität für die Bindung von Biomolekülen steigt. Weiterhin ist bekannt, dass die Polyelektrolytschichten der Kontur der Oberfläche des inerten Trägers folgen, da die Polymerkette des Polyelektrolyts an sich sehr flexibel ist. Ohne an diese Theorie gebunden sein zu wollen, gehen die Erfinder davon aus, dass auf der Oberfläche des inerten Trägers bereits solche Poren von der aus den Polyelektrolyten gebildeten Schicht ausgekleidet werden, die einen Durchmesser von mehr als 10 nm aufweisen, sodass sich eine Vielzahl von Koordinationsstellen für die Immobilisierung von Biomolekülen ergeben. Ferner bildet sich durch die Doppelschicht bzw. Mehrfachschicht aus entgegengesetzt geladenen Polyelektrolyten eine membranartige, nanoporöse Struktur aus. Es ist auch denkbar, dass sich in eine derartige aus Polyelektrolyten aufgebaute Schicht Biomoleküle einlagern können, sofern deren Molekülgewicht nicht zu hoch gewählt wird.

Erfindungsgemäß wird daher ein Trägermaterial bereitgestellt, welches zumindest umfasst:
a.) einen inerten Trägerkern, welcher eine Oberfläche mit einer positiven oder negativen Gesamtladung aufweist;
b.) eine auf dem inerten Trägerkern angeordnete und sich an diesen anschließende erste Schicht aus einem ersten Polyelektrolyt, welcher eine zur Oberflächenladung des inerten Trägerkerns gleichsinnige Gesamtladung aufweist;
c.) eine auf der ersten Schicht aus einem ersten Polyelektrolyt angeordnete zweite Schicht aus einem zweiten Polyelektrolyt, welcher eine zur Gesamtladung des ersten Polyelektrolyts gegensinnige Gesamtladung aufweist.

Das Trägermaterial umfasst zunächst einen inerten Trägerkern. Das Material des Trägerkerns ist zunächst keinen besonderen Anforderungen unterworfen. Es können sowohl anorganische als auch organische Materialien verwendet werden. Der inerte Trägerkern sollte in Lösungsmitteln, die für die Herstellung bzw. die spätere Anwendung des Trägermaterials verwendet werden, unlöslich sein und keine Reaktion mit diesen eingehen. Ebenso sollte sich der Trägerkern bevorzugt inert gegenüber einer Reaktion mit dem Polyelektrolyt sowie den ggf. auf dem Trägermaterial immobilisierten Biomolekülen zeigen, also insbesondere keine kovalente Bindung zu diesen Materialien ausbilden. Der inerte Trägerkern trägt eine positive oder negative Gesamtladung, wobei diese Gesamtladung in erster Linie durch die auf der Oberfläche des inerten Trägerkerns vorhandenen Ladungen bestimmt wird. Die Gesamtladung des inerten Trägerkerns lässt sich am besten bestimmen, bevor dieser mit dem Polyelektrolyt umgesetzt bzw. beladen wird.

Die auf der Oberfläche des inerten Trägerkerns vorhandene Ladung lässt sich beispielsweise durch eine Bestimmung des Zetapotentials charakterisieren. Das Zetapotential ist abhängig vom pH-Wert der Lösung, mit der die Grenzfläche des Festkörpers oder des Partikels in Kontakt steht, der Art des eingesetzten Elektrolyts sowie der Konzentration des Elektrolyts. Experimentell lässt sich das Zetapotential beispielsweise durch elektrokinetische Messungen bestimmen. Eine häufig verwendete Methode ist die Elektrophorese, bei welcher die Wanderungsgeschwindigkeit der Teilchen im elektrischen Feld zur Bestimmung des Zetapotentials herangezogen wird. Die Meßmethode ist beispielsweise in G. Lagaly, O. Schulz, R. Zimehl; "Dispersionen und Emulsionen, eine Einführung in die Kolloidik fein verteilter Teilchen einschließlich der Tonminerale", Kapitel 10, "Oberflächenpotential und elektrokinetisches Potential", S. 347 ff beschrieben.

Das Zetapotential des inerten Trägerkerns beträgt unter den Beschichtungsbedingungen für negativ geladene Trägermaterialien dem Betrag nach bevorzugt mindestens 1 mV, besonders bevorzugt mindestens 2,5 mV, ganz besonders bevorzugt zumindest 10 mV. Gemäß einer Ausführungsform beträgt das Zetapotential weniger als 150 mV. Analog beträgt das Zetapotential für die positiv geladenen Trägermaterialien dem Betrag nach bevorzugt mindestens 1 mV, besonders bevorzugt mindestens 2,5 mV, ganz besonders bevorzugt mindestens 5 mV. Gemäß einer Ausführungsform beträgt das Zetapotential weniger als 150 mV.

Eine weitere Größe zur Charakterisierung des Materials des inerten Trägerkerns ist der Ladungsnullpunkt der Oberfläche, "Point of Zero Charge" (PZC). Der Ladungsnullpunkt entspricht dem pH Wert, bei dem die Ladung (somit auch das Potential) der Grenzfläche des Trägerkerns null ist. Bei oxidischen Materialien lässt sich der PZC beispielsweise durch eine Säure-Base Titration bestimmen, wobei zusätzlich verschiedene Elektrolytkonzentrationen eingestellt werden. Die Details zur Meßmethode sind beschreiben in: G. Lagaly, O. Schulz, R. Zimehl, Dispersionen und Emulsionen, Eine Einführung in die Kolloidik fein verteilter Stoffe einschließlich der Tonminerale, Kapitel 9 : Oberflächenladung S. 322 ff. Man geht davon aus, dass die Grenzfläche des Festkörpers bei pH Werten unterhalb seines PZC positiv und bei pH-Werten oberhalb seines PZCs negativ geladen ist. Eine Zusammenstellung von Daten zu den Ladungsnullpunkten verschiedener Materialien findet sich beispielsweise in: Marek Kosmulski, Chemical Properties of Material Surfaces, Surfactant Science Series volume 102, Marcel Dekker, New York, Basel, 2001, PZCs of simple (Hydr)oxides, S. 89ff.

Negativ geladene Träger im Rahmen der Erfindung sind solche, bei denen der pH-Wert, bei dem die Polyelektrolytbeschichtung durchgeführt wird, oberhalb des PZC's des Trägers liegt. Positiv geladene Träger im Rahmen der Erfindung sind solche, bei denen der pH-Wert, bei dem die Polyelektrolytbeschichtung durchgeführt wird, unterhalb des PZC's liegt. Einige Trägermaterialien können deshalb, abhängig von dem pH-Wert, bei dem die Beschichtung durchgeführt wird, sowohl als negativ geladene Träger, als auch als positiv geladene Träger eingesetzt werden.

Auf dem inerten Trägerkern ist eine erste Schicht aus einem ersten Polyelektrolyt aufgebracht. Unter einem Polyelektrolyt wird eine Verbindung verstanden, die mehrere geladene oder stark polarisierte Gruppen aufweist und die daher zur Ausbildung ionischer oder stark dipolarer Bindungen befähigt ist. Unter einer stark dipolaren Gruppe wird eine Gruppe verstanden, die beispielsweise Wasserstoffbrückenbindungen ausbilden kann, jedoch noch nicht in ionischer Form vorliegt. Beispiele für derartige Gruppen sind sauerstoff- sowie stickstoffhaltige Gruppen, welche eine polare Bindung zu einem Wasserstoffatom ausbilden können. Die Anzahl der geladenen bzw. stark polarisierten Gruppen beträgt zumindest drei. Bevorzugt ist aber eine deutlich höhere Anzahl an geladenen bzw. stark polarisierten Gruppen im Polyelektrolyt vorgesehen. Bevorzugt umfasst der Polyelektrolyt zumindest 5, bevorzugt zumindest 10, besonders bevorzugt zumindest 50 geladene oder stark dipolare Gruppen. Um eine Belegung des inerten Trägerkerns zu erhalten, ist es an sich ausreichend, wenn der Polyelektrolyt weniger als 20000, bevorzugt weniger als 15000 geladene oder stark dipolare Gruppen aufweist. Es ist jedoch auch möglich, Polyelektrolyte einzusetzen, die eine höhere Anzahl an geladenen oder stark dipolaren Gruppen aufweisen. Der Polyelektrolyt wird bevorzugt von einem Polymer gebildet, welches geladene bzw. stark polarisierte Gruppen trägt. Derartige Polymere lassen sich mit üblichen Polymerisationsreaktionen aus geeigneten Monomeren herstellen, welche bereits die geladenen bzw. stark polarisierten Gruppen tragen, ggf. auch in geschützter Form. Alternativ können auch Polymere verwendet werden, die aus biologische Quellen erhalten wurden. Beispiele für geeignete Biomoleküle sind Chitosane, Alginate, Carboxymethylcellulose etc.

Der erste Polyelektrolyt weist eine zur Gesamtladung des inerten Trägers gleichsinnige Ladung auf. Weist der inerte Träger eine positive Gesamtladung auf, so wird ein erster Polyelektrolyt gewählt, der ebenfalls eine positive Gesamtladung trägt. Weist der inerte Trägerkern hingegen eine negative Gesamtladung auf, so wird ein erster Polyelektrolyt gewählt, der ebenfalls eine negative Gesamtladung aufweist. Dabei sollen die jeweiligen Gesamtladungen das gleiche Vorzeichen aufweisen. Es ist jedoch nicht erforderlich, dass die Gesamtladungen auch dem Betrag nach gleich sind.

Das Vorzeichen der Gesamtladung des ersten Polyelektrolyts lässt sich meist bereits aus der Ladung der Gruppen abschätzen, die am ersten Polyelektrolyt gebunden sind. Das Vorzeichen der Gesamtladung lässt sich aber auch bestimmen, indem die elektrophoretische Mobilität gemessen wird. Wandert der Polyelektrolyt unter dem Einfluss des elektrischen Feldes zur Anode, weist er eine negative Gesamtladung auf. Wandert der Polyelektrolyt zur Kathode, so weist er eine positive Gesamtladung auf.

Die erste Schicht aus dem ersten Polyelektrolyt ist bevorzugt nur über physikalische Bindungen an die Oberfläche des inerten Trägers gebunden, also über ionische oder polare Bindungen, und nicht über kovalente Bindungen. Eine Bindung über kovalente Bindungen ist jedoch denkbar, wenn bei der Herstellung des Trägermaterials ein geeignetes bifunktionelles Vernetzungsagens verwendet wird, welches eine kovalente Vernetzung von erstem Polyelektrolyt und inertem Trägerkern ermöglicht.

Auf der ersten Schicht aus dem ersten Polyelektrolyt ist eine zweite Schicht angeordnet, die aus einem zweiten Polyelektrolyt gebildet ist. Der zweite Polyelektrolyt weist eine Gesamtladung auf, deren Vorzeichen entgegengesetzt zum Vorzeichen der Gesamtladung des ersten Polyelektrolyts ist. Weist der erste Polyelektrolyt also eine positive Gesamtladung auf, so weist der zweite Polyelektrolyt eine negative Gesamtladung auf. Weist der erste Polyelektrolyt dagegen eine negative Gesamtladung auf, so weist der zweite Polyelektrolyt eine positive Gesamtladung auf. Wie bereits oben beschrieben gilt auch hier, dass die Gesamtladung des ersten Polyelektrolyts und die Gesamtladung des zweiten Polyelektrolyts zwar entgegengesetzte Vorzeichen haben. Es ist aber nicht erforderlich, dass die Gesamtladungen dem Betrag nach gleich sind.

Als inerter Trägerkern wird bevorzugt ein Material gewählt, welches eine hohe spezifische Oberfläche aufweist, sodass beim erfindungsgemäßen Trägermaterial eine aus Polyelektrolyten entgegengesetzter Gesamtladung gebildete Mehrfachschicht bereitgestellt wird, welche ebenfalls eine hohe Oberfläche für die Bindung von Biomolekülen bereitstellt. Bevorzugt weist der inerte Trägerkern eine spezifische Oberfläche von zumindest 0,7 m²/g, besonders bevorzugt zumindest 1 m²/g, insbesondere bevorzugt zumindest 10 m²/g auf. Gemäß einer Ausführungsform ist die spezifische Oberfläche des inerten Trägerkerns größer als 50 m²/g. Gemäß einer weiteren Ausführungsform ist die spezifische Oberfläche des inerten Trägerkerns geringer als 600 m²/g gewählt. Die spezifische Oberfläche des inerten Trägers kann mit Hilfe von Stickstoffporosimetrie bestimmt werden. Das Verfahren ist bei den Beispielen genauer beschrieben.

Nach den Vorstellungen der Erfinder sind zumindest die an der Oberfläche des inerten Trägers angeordneten Poren mit der aus Polyelektrolyten gebildeten Mehrfachschicht ausgekleidet. Die so vorbereiteten Poren können beispielsweise als geschützte Reaktionsräume für eine enzymatisch katalysierte Reaktion wirken. Bevorzugt weist der inerte Trägerkern ein Gesamtporenvolumen von zumindest 10 mm³/g, besonders bevorzugt zumindest 50 mm³/g, insbesondere zumindest 100 mm³/g auf. Gemäß einer Ausführungsform weist der inerte Trägerkern ein Porenvolumen von weniger als 700 mm³/g auf. Die Angabe des Gesamtporenvolumens bezieht sich dabei auf das kumulative Porenvolumen für Poren mit einem Durchmesser von 1,7 bis 300 nm, ermittelt nach der BJH-Methode (E.P. Barret, L.G. Joiner, P.P. Haienda, J. Am. Chem. Soc. 73 (1951), 373).

Bevorzugt werden als inerter Trägerkern poröse Materialien verwendet, die eine hohe Porosität aufweisen. Bevorzugt werden mehr als 90 % des Porenvolumens des inerten Trägerkerns von Poren gebildet, die einen Porendurchmesser von mindestens 5 nm, bevorzugt von 10 bis 100 nm aufweisen.

Der inerte Trägerkern weist bevorzugt Poren einer Größe auf, dass diese von der aus den Polyelektrolyten ausgebildeten Schicht ausgekleidet werden können. Die Poren sollten jedoch nicht von den auf dem Trägermaterialien adsorbierten Biomolekülen verstopft werden. Der mittlere Porendurchmesser D₅₀, bestimmt mit der BJH-Methode, des inerten Trägers wird daher bevorzugt zwischen 5 und 200 nm, besonders bevorzugt zwischen 10 und 100 nm gewählt. Der mittlere Porendurchmesser D₅₀ bedeutet dabei, dass 50 % der Poren einen größeren Durchmesser und 50 % der Poren einen kleineren Durchmesser aufweisen.

Ein Verfahren zur Bestimmung des Porenvolumens sowie der Porenverteilung ist bei den Beispielen angegeben.

Bevorzugt weist der inerte Trägerkern eine relativ kleine Partikelgröße auf, sodass die für Biomoleküle oder Reaktionsstoffe zugängliche Oberfläche des Trägermaterials groß ist, also eine große Menge an Biomolekülen gebunden werden kann. Ebenso ist eine kleine Partikelgröße vorteilhaft, wenn beispielsweise ein mit einem Enzym beladenes Trägermaterial in einer enzymatisch katalysierten Reaktion eingesetzt wird, da dies den Transport der Ausgangsmaterialien bzw. der Produkte zum bzw. vom Enzym erleichtert. Wird die Partikelgröße jedoch zu gering gewählt, können beispielsweise Probleme bei der Abtrennung des Trägermaterials von einem Reaktionsmedium auftreten. Der inerte Trägerkern weist bevorzugt eine Partikelgröße von zumindest 10 µm, besonders bevorzugt zumindest 30 µm, weiter bevorzugt zumindest 40 µm, und gemäß einer weiteren Ausführungsform zumindest 50 µm auf. Bevorzugt wird die Partikelgröße des inerten Trägers kleiner als 2000 µm, besonders bevorzugt kleiner als 1000 µm gewählt. Ein Verfahren zur Bestimmung der Partikelgröße ist bei den Beispielen angegeben.

Wie bereits erläutert, weist der inerte Trägerkern eine positive oder negative Gesamtladung auf. Die Gesamtladung sowie das Vorzeichen der Gesamtladung des Trägerkerns hängen auch von den Bedingungen ab, die bei der Beschichtung des inerten Trägerkerns mit dem Polyelektrolyt verwendet werden. Bevorzugt weist der inerte Trägerkern unter den Bedingungen, welche bei der Beschichtung des inerten Trägerkerns mit dem Polyelektrolyt eingestellt werden, ein Zetapotential auf, dessen Betrag größer als 1 mV ist. Bevorzugt werden jedoch Trägerkerne verwendet, die eine höhere Ladung aufweisen. Bevorzugt weist der inerte Trägerkern eine Ladung mit einem Betrag von mehr als 2,5 mV, besonders bevorzugt von mehr als 5 mV auf. Ein Verfahren zur Bestimmung der Ladung des Trägerkerns ist bei den Beispielen angegeben.

Wie bereits erläutert, kann der inerte Trägerkern an sich aus jedem Material bestehen, dass sich unter den Anforderungen bei der Herstellung oder dem Gebrauch des Trägermaterials inert verhält. So kann der inerte Trägerkern gemäß einer Ausführungsform aus einem organischen Polymer gebildet sein. Derartige Polymere lassen sich, beispielsweise durch Quervernetzung, auch in Form kleiner poröser Kugeln herstellen. Geeignete Polymere sind beispielsweise Polystyrol, Polymethylmethacrylat, Melamin-Formaldehydharze, vernetzte Dextrane oder vernetztes Chitosan.

Gemäß einer weiteren Ausführungsform ist der inerte Trägerkern aus einem anorganischen Material aufgebaut. Solche anorganischen Materialien können einerseits relativ einfach in großen Mengen und zu günstigen Kosten bereitgestellt werden und andererseits auch in ihren Eigenschaften, insbesondere der spezifischen Oberfläche, Porosität und Oberflächenladung relativ genau eingestellt werden.

Bevorzugte anorganische Materialien sind ausgewählt aus der Gruppe von Siliziumdioxid, Aluminiumoxid, Boehmit, Zeolithen, Titandioxid, Zirkondioxid, Zinnoxid, Tonen, insbesondere wärmebehandelten Tonmineralien, sowie Silicate. Daneben sind auch poröse Glaspartikel als Träger geeignet.

Werden wärmebehandelte Tonmineralien verwendet, so wird die Hitzebehandlung bevorzugt in der Weise durchgeführt, dass die Quellfähigkeit des hitzebehandelten Tonmaterials in Wasser weniger als 15 ml/2g, vorzugsweise weniger als 10 ml/2g beträgt und damit im Wesentlichen dem Sedimentvolumen entspricht. Bevorzugt wird für die Wärmebehandlung eine möglichst niedrige Temperatur gewählt und gegebenenfalls die Dauer der Temperaturbehandlung angepasst. Bevorzugt werden für die Wärmebehandlung Temperaturen von zumindest 400°C, bevorzugt zumindest 450°C, insbesondere bevorzugt zumindest 500°C gewählt. Das als Ausgangsmaterial verwendete Tonmaterial sollte jedoch auch keine zu hohe thermische Belastung erfahren, da sonst die Adsorptionskapazität für Biomoleküle, wie beispielsweise Proteine, absinkt. Bevorzugt werden die Temperaturen daher niedriger als 1000°C, vorzugsweise niedriger als 800°C und insbesondere bevorzugt unterhalb von 700°C gewählt. Die Dauer der Behandlung ist abhängig von der Menge des eingesetzten Tonmaterials sowie vom Ofentyp. Bevorzugt werden Drehrohröfen für die Wärmebehandlung verwendet, da diese ein gleichmäßiges Erhitzen des Rohtonmaterials ermöglichen. Die Behandlungsdauer wird vorzugsweise möglichst kurz gewählt, um eine übermäßige thermische Belastung des Rohtonmaterials zu vermeiden. Bei einer industriellen Durchführung des Verfahrens werden Behandlungszeiten von zumindest 10 Minuten, vorzugsweise zumindest 20 Minuten gewählt. Um eine thermische Überbelastung zu vermeiden, wird die Behandlungszeit bevorzugt kürzer als 2 Stunden, insbesondere bevorzugt kürzer als 1 Stunde gewählt. Die geeignete Behandlungsdauer kann vom Fachmann leicht ermittelt werden, indem während der thermischen Behandlung des Rohtons regelmäßig Proben entnommen und auf ihre Quellfähigkeit untersucht werden.

Siliziumdioxid kann beispielsweise in Form von Kieselgel oder Fällungskieselsäure bereitgestellt werden. Smektitische Tonmineralien werden bevorzugt in wärmebehandelter Form bereitgestellt, da diese Tonmaterialien dann nur noch eine sehr geringe Neigung zum Quellen in Wasser aufweisen.

Besonders bevorzugt werden Aluminiumsilikate und hier insbesondere bevorzugt schichtförmige Aluminiumsilikate verwendet. Aluminiumsilikate weisen an ihrer Oberfläche Inhomogenitäten in der Ladungsverteilung auf. Es steht daher eine ausreichende Anzahl von Bindungsstellen für den ersten Polyelektrolyt zur Verfügung. Schichtförmige Aluminiumsilikate neigen in wässrigen Systemen zum Quellen. Bevorzugt werden daher Tonmineralien verwendet, die einer Wärmebehandlung unterzogen wurden, sodass sie in Wasser nicht mehr oder nur in sehr geringem Maß quellen.

Bevorzugt können auch reine Kieselgele verwendet werden, die mit sehr engen Porengrößenverteilungen verfügbar sind. Solche Kieselgele werden beispielsweise unter der Bezeichnung Davisil^{®} Grade 635 von der Firma Sigma-Aldrich angeboten. Dieses Kieselgel weist eine Korngröße im Bereich von 35 bis 75 µm, eine Porengröße von 60 Å, ein Porenvolumen von 0,75 ml/g sowie eine spezifische Oberfläche von 480 m²/g auf. Vergleichbare Kieselgele werden unter der Bezeichnung Lichrosorb^{®} Si100 oder Merck DC Grade 11678 von der Merck KgaA, Darmstadt, DE angeboten. Weiter ist auch Calciumcarbonat als Trägerkern geeignet. Es kann sowohl Calciumcarbonat aus natürlichen Quellen als auch synthetisches Material verwendet werden.

Die Erfinder nehmen an, ohne an diese Theorie gebunden sein zu wollen, dass bei der Herstellung des Trägermaterials der erste Polyelektrolyt in der Lösung in Form eines Knäuels vorliegt. Die Konformation der Polymerkette kann beispielsweise durch die Salzkonzentration beeinflusst werden, die in der Lösung des Polymers eingestellt wird. Dieser Knäuel kann in Poren des inerten Trägerkerns eindringen und sich dann entfalten und an der Wand anlagern, sodass die Wand der Pore mit dem Polyelektrolyt ausgekleidet wird. Um ein Eindringen des Polyelektrolyts in Poren des inerten Trägers und eine Entfaltung des Polyelektrolyts in der Pore zu ermöglichen, wird das Molekülgewicht des ersten Polyelektrolyts bevorzugt nicht zu hoch gewählt. Bevorzugt weist der erste Polyelektrolyt ein Molekülgewicht von weniger als 100.000 g/mol, besonders bevorzugt weniger als 70.000 g/mol, insbesondere bevorzugt weniger als 50.000 g/mol auf. Um eine gleichmäßige Beschichtung der Oberfläche des inerten Trägers zu erreichen, weist der erste Polyelektrolyt bevorzugt ein Molekülgewicht von mehr als 1.000 g/mol, besonders bevorzugt mehr als 5.000 g/mol auf.

Das Molekülgewicht des Polyelektrolyts lässt sich durch Standardverfahren, wie Lichtstreuung, Ultrazentrifugation oder Größenausschlusschromatographie bestimmen. Ein geeignetes Verfahren ist bei den Beispielen beschrieben.

Um auf der Oberfläche des inerten Trägerkerns aufziehen zu können, sollte der erste Polyelektrolyt einerseits geladene oder polare Gruppen aufweisen, die ionische oder polare Bindungen zum inerten Träger ausbilden können. Andererseits sollte der erste Polyelektrolyt auch eine ausreichende Flexibilität aufweisen, sodass er sich an die auf der Oberfläche des inerten Trägerkerns vorliegende Ladungsverteilung strukturell anpassen kann. Vorzugsweise weist der erste Polyelektrolyt geladene Wiederholungseinheiten auf, welche eine kationische und/oder anionische Gruppe tragen, wobei der Anteil der geladenen Wiederholungseinheiten mehr als 20 mol-%, bevorzugt mehr als 40 mol-%, besonders bevorzugt mehr als 60 mol-% beträgt, bezogen auf die Gesamtheit der Wiederholungseinheiten. Es kann jede Wiederholungseinheit eine kationische und/oder anionische Gruppe tragen. Gemäß einer Ausführungsform beträgt der Anteil der Wiederholungseinheiten, welche eine anionische oder kationische Gruppe tragen, weniger als 80 mol-%, gemäß einer weiteren Ausführungsform weniger als 70 mol-%, jeweils bezogen auf die Gesamtzahl der Wiederholungseinheiten. Eine Wiederholungseinheit entspricht dabei dem Äquivalent eines Monomers, welches bei der Herstellung des Polyelektrolyts in die Polymerkette eingebaut wird. Bevorzugt trägt die geladene Wiederholungseinheit nur eine anionische bzw. kationische Gruppe. Es ist aber auch möglich, mehrere anionische bzw. kationische Gruppen an einer geladenen Wiederholungseinheit vorzusehen. Beispielhafte kationische Gruppen sind protonierbare stickstoffhaltige Gruppen, wie primäre, sekundäre oder tertiäre Aminogruppen, sowie quaternäre Ammoniumgruppen. Beispielhafte anionische Gruppen sind Carboxylgruppen, Sulfonsäuregruppen, Phosphatgruppen, Phosphonatgruppen, Sulfatgruppen und Boronsäuregruppen. Sofern ein Polyelektrolyt in verschiedenen Ladungszuständen auftreten kann, also in Abhängigkeit von den herrschenden Bedingungen sowohl eine positive Gesamtladung als auch eine negative Gesamtladung aufweisen kann, wird das Vorzeichen der Gesamtladung des Polyelektrolyts bei den Bedingungen bestimmt, bei welchen der Polyelektrolyt auf den ,ggf. vorbeschichteten, inerten Trägerkern aufgebracht wird. Eine solche Änderung des Vorzeichens der Gesamtladung des Polyelektrolyts kann beispielsweise durch eine Änderung des pH-Wertes verursacht werden.

Gemäß einer ersten Ausführungsform ist der erste Polyelektrolyt ausgewählt aus kationischen stickstoffhaltigen Polymeren. Die kationischen stickstoffhaltigen Gruppen des kationischen stickstoffhaltigen Polymers können auch dadurch erhalten werden, dass das Stickstoffatom dieser Gruppen in protonierter Form vorliegt. Das Stickstoffatom ist in diesen Gruppen bevorzugt in sp³-hybridisierter Form enthalten, also in Form einer Aminogruppe, eines Ammoniumsalzes oder einer Iminogruppe. Es ist jedoch auch denkbar, dass der Stickstoff in sp²-hybridisierter Form vorliegt, beispielsweise als Heteroatom in einer heteroaromatischen Gruppe, wie Polyvinylpyridin.

Bevorzugt ist das kationische stickstoffhaltige Polymer ausgewählt aus der Gruppe von Polyaminen, Polyiminen, Polypyridinen und Polyimidazolen. Geeignet sind auch kationische stickstoffhaltige Polymere, die aus biologischen Quellen gewonnen werden, sowie Derivate dieser Verbindungen. Ein beispielhaftes Biopolymer ist Chitosan.

Weiter bevorzugt trägt das kationische stickstoffhaltige Polymer primäre, sekundäre und/oder tertiäre Aminogruppen und/oder quartäre Ammoniumgruppen. Das kationische stickstoffhaltige Polymer kann nur gleichartige Aminogruppen umfassen, beispielsweise nur primäre, sekundäre oder tertiäre Aminogruppen bzw. quaternäre Ammoniumgruppen. Es ist aber auch möglich, Kombinationen verschiedener stickstoffhaltiger Gruppen am kationischen stickstoffhaltigen Polymer vorzusehen. Die Substituenten an verschiedenen stickstoffhaltigen Gruppen können gleich oder verschieden sein. Weiter können die stickstoffhaltigen Gruppen seitenständig zur Polymerhauptkette angeordnet sein. Es ist aber auch möglich, Stickstoffatome als Teil der Polymerhauptkette vorzusehen.

Gemäß einer Ausführungsform ist das kationische stickstoffhaltige Polymer ausgewählt aus der Gruppe von Polydiallyldimethylammonium-, Polyacryloxyethyltrimethylammonium- und Polyacryloxybenzyltrimethylammoniumsalzen, Polyallylammoniumsalzen und den Chitosanen. Bevorzugt werden die Salze durch die protonierte Form der kationischen stickstoffhaltigen Polymere gebildet.

Wie bereits erläutert, kann das Stickstoffatom auch in der Polymerhauptkette des kationischen stickstoffhaltigen Polymers enthalten sein. Gemäß einer Ausführungsform ist das kationische stickstoffhaltige Polymer ein Polyethylenimin.

Gemäß einer weiteren Ausführungsform ist der erste Polyelektrolyt von einem anionischen Polymer gebildet, welches eine negative Gesamtladung aufweist. Ein derartiger negativ geladener erster Polyelektrolyt ist also auf einem inerten Trägerkern angeordnet, der selbst eine negative Gesamtladung trägt. Ein negativ geladener Polyelektrolyt erhält seine Ladung durch negativ geladene Gruppen, die seitenständig zur Polymerhauptkette angeordnet sein können oder auch einen Teil der Polymerhauptkette bilden können. Das Vorzeichen der Gesamtladung lässt sich aus dem Vorzeichen der geladenen bzw. polarisierten Gruppen abschätzen, die im ersten Polyelektrolyt enthalten sind oder beispielsweise auch durch Messung der elektrophoretischen Beweglichkeit quantifizieren. Die anionischen Gruppen werden meist bei der Synthese des anionischen Polymers über die als Ausgangsmaterial eingesetzten Monomere in das Polymer eingeführt. Die anionischen Gruppen können dabei ggf. auch in geschützter Form vorliegen und erst nach der Synthese des Polymers freigesetzt werden. Es ist aber auch möglich, die anionischen Gruppen erst nach der Synthese des Polymers in das Polymer einzuführen, um dadurch ein anionisches Polymer zu erhalten.

Sofern sich die Ladung des Polyelektrolyts nicht bereits aus seiner Struktur eindeutig abschätzen lässt, kann die Ladung mit Hilfe der Gelektrophorese bestimmt werden.

Geeignete anionische Gruppen für den ersten Polyelektrolyt sind beispielsweise Carboxylgruppen, Sulfonsäuregruppen, saure Hydroxylgruppen, Phosphatgruppen, Phosphonatgruppen, Sulfatgruppen und Boronsäuregruppen.

Gemäß einer Ausführungsform ist das anionische Polymer ausgewählt aus der Gruppe von Polycarbonsäuren, Polysulfonsäuren, Polyboronsäuren, Polyphosphaten, Polyphosphonaten und Polysulfaten.

Bevorzugt ist das anionische Polymer ausgewählt aus der Gruppe von Polystyrolsulfonat, Poly(meth)acrylat, Copolymeren von Styrol oder Styrolsulfonsäure und Maleinsäure.

Auf die auf dem inerten Trägerkern angeordnete Schicht aus dem ersten Polyelektrolyt ist eine zweite Schicht aus einem zweiten Polyelektrolyt angeordnet, wobei erster Polyelektrolyt und zweiter Polyelektrolyt entgegengesetzt geladen sind. Dabei weisen die Gesamtladungen von erstem Polyelektrolyt und zweitem Polyelektrolyt ein entgegengesetztes Vorzeichen auf. Es ist jedoch nicht erforderlich, dass die Gesamtladungen von erstem Polyelektrolyt und zweitem Polyelektrolyt dem Betrag nach gleich sind. Auch der zweite Polyelektrolyt liegt bevorzugt in Form eines Polymers vor, welches geladene Gruppen aufweist. Wie beim ersten Polyelektrolyt wird auch beim zweiten Polyelektrolyt das Molekülgewicht bevorzugt innerhalb bestimmter Grenzen gewählt, um einerseits eine stabile Fixierung der Schicht des zweiten Polyelektrolyts auf der Schicht des ersten Polyelektrolyts zu erreichen und andererseits ein Abdecken der Porenöffnung und damit ein Verschließen auch der größeren Poren zu verhindern. Bevorzugt weist der zweite Polyelektrolyt ein Molekülgewicht im Bereich von 5.000 bis 5.000.000 g/mol, besonders bevorzugt 15.000 bis 1.000.000 g/mol, insbesondere 50.000 bis 300.000 g/mol auf.

Gemäß einer ersten Ausführungsform ist der zweite Polyelektrolyt ein anionisches Polymer, welches eine negative Gesamtladung aufweist. Bei dieser Ausführungsform besitzt der inerte Trägerkern also eine positive Gesamtladung, der erste Polyelektrolyt ebenfalls eine positive Gesamtladung und der zweite Polyelektrolyt eine negative Gesamtladung.

Als anionisches Polymer können an sich alle anionischen Polymere verwendet werden, die bereits beim ersten Polyelektrolyt diskutiert wurden. Es wird daher auf die entsprechenden Abschnitte verwiesen.

Bevorzugt ist das anionische Polymer ausgewählt aus der Gruppe von Polystyrolsulfonat, Poly(meth)acrylat, Copolymeren von Styrol oder Styrolsulfonsäure, und Maleinsäure. Weitere bevorzugte anionische Polymere sind Polymere aus biologischen Quellen sowie deren Derivate. Beispiel für derartige Biopolymere sind Alginate, Carrageenan, Chondroitinsulfat, Carboxymethylcellulose, Sulfoethylcellulose, Dextransulfat. Mit solchen Biopolymeren können auch chirale Funktionen in die Polyelektrolytschicht eingebracht werden.

Gemäß einer zweiten Ausführungsform ist der zweite Polyelektrolyt ein kationisches Polymer, welches eine positive Gesamtladung aufweist. Bei dieser Ausführungsform weist also der inerte Trägerkern eine negative Gesamtladung auf, der erste Polyelektrolyt ebenfalls eine negative Gesamtladung und der auf dem ersten Polyelektrolyt angeordnete zweite Polyelektrolyt eine positive Gesamtladung.

Als kationisches Polymer können an sich die bereits beim ersten Polyelektrolyt beschriebenen kationischen Polymeren verwendet werden.

Bevorzugt ist das kationische stickstoffhaltige Polymer ausgewählt aus der Gruppe von Polyaminen, Polyiminen und Polyimidazolen. Besonders bevorzugt werden Polymere aus biologischen Quellen sowie deren Derivate verwendet. Ein geeignetes Biopolymer ist beispielsweise Chitosan. Diese Salze liegen bevorzugt als protonierte Form der genannten Polymere vor.

Das Trägermaterial kann neben der auf dem inerten Trägerkern angeordneten ersten Schicht aus dem ersten Polyelektrolyt und der auf der ersten Schicht aus dem ersten Polyelektrolyt angeordneten zweiten Schicht aus dem zweiten Polyelektrolyt noch weitere Schichten umfassen, die aus einem Polyelektrolyt gebildet sind.

Gemäß einer Ausführungsform ist auf der zweiten Schicht aus dem zweiten Polyelektrolyt zumindest eine weitere Schicht aus einem Polyelektrolyt angeordnet, wobei die Polyelektrolyte aufeinander angeordneter Schichten eine gegensinnige Gesamtladung aufweisen.

Weist die zweite Schicht des Polyelektrolyts also eine positive Gesamtladung auf, so ist auf dieser eine Schicht eines weiteren Polyelektrolyts angeordnet, wobei dieser eine negative Gesamtladung aufweist. Weist die zweite Schicht des Polyelektrolyts dagegen eine negative Gesamtladung auf, so weist der weitere Polyelektrolyt eine positive Gesamtladung auf. Als weiterer Polyelektrolyt können die bereits beschriebenen Polyelektrolyten verwendet werden.

Auf der Oberfläche des inerten Trägerkerns ist also eine Hülle aus zumindest zwei Schichten aufgetragen, die aus gegensinnig geladenen Polyelektrolyten aufgebaut sind. Diese Hülle lässt sich durch Auftragen weiterer Schichten aus Polyelektrolyt verdicken, wobei ein Stapel aus Schichten von gegensinnig geladenen Polyelektrolyten gebildet wird. Die Schichtdicke dieser Hülle lässt sich an sich durch den Auftrag weiterer Schichten aus Polyelektrolyt vergrößern. Die Anzahl der Polyelektrolytschichten ist jedoch vorzugsweise auf einen Wert beschränkt, der durch die Anforderung bestimmt wird, dass die Poren des inerten Trägers zumindest teilweise mit der Schicht aus den Polyelektrolyten gefüllt sind, sodass die für die Adsorption von beispielsweise Biomolekülen zur Verfügung stehenden Fläche möglichst groß bleibt.

Gemäß einer Ausführungsform ist die Anzahl der weiteren Schichten aus einem Polyelektrolyt geringer als drei gewählt. Die Gesamtzahl der auf dem inerten Trägerkern aufgebrachten Schichten aus Polyelektrolyt kann vorzugsweise also maximal fünf betragen. Vorzugsweise beträgt die Anzahl der auf dem inerten Trägerkern aufgebrachten Polyelektrolytschichten weniger als 4, besonders bevorzugt weniger als 3.

Um die Stabilität der aus Polyelektrolytschichten gebildeten, auf dem inerten Träger angeordneten Hülle zu verbessern, können die Polyelektrolytschichten gemäß einer Ausführungsform auch kovalent vernetzt sein. Die Vernetzung kann sowohl innerhalb einer Polyelektrolytschicht vorgesehen sein als auch zwischen verschiedenen Polyelektrolytschichten. Die erste Polyelektrolytschicht kann auch mittels geeigneter Moleküle mit dem inerten Trägerkern vernetzt sein. Als Vernetzungsagenzien können übliche, zumindest bifunktionelle Verbindungen verwendet werden. Zwischen den reaktiven Gruppen dieser bifunktionellen Verbindungen ist vorzugsweise ein Abstand vorgesehen, sodass die Distanz zwischen entsprechenden Gruppen an den Polyelektrolyten überbrückt werden kann. Geeignete bifunktionelle Verbindungen sind beispielsweise Dialdehyde, wie Glutaraldehyd oder Malondialdehyd zur Vernetzung eines kationischen Polyelektrolyts.

Zur Vernetzung anionischer Polyelektrolyte, welche Carboxygruppen enthalten eignen sich, bevorzugt nach einer Aktivierung durch Carbodiimide, beispielsweise Di- bzw. Polyamine, wie Ethylendiamin oder Hydrazin.

Bevorzugt wird jedoch keine Vernetzung der Polyelektrolytschichten durchgeführt, da bei der Vernetzung geladene Gruppen des Polyelektrolyts verbraucht werden und daher nicht mehr für eine Koordination einer weiteren Polyelektrolytschicht bzw. von Biomolekülen zur Verfügung stehen. Gemäß einer Ausführungsform ist lediglich die äußere Polyelektrolytschicht des Trägermaterials kovalent vernetzt.

Die Vernetzung wird mit üblichen Verfahren durchgeführt. Beispielsweise kann eine Vernetzung zwischen einer Polyanionenschicht, in welcher Carboxylgruppen enthalten sind, und einer Polykationenschicht, welche primäre oder sekundäre Aminogruppen enthält, durch eine einfache thermische Nachbehandlung oder durch Zugabe eines Carbodiimids erreicht werden. Die Vernetzung einer kationischen Polyelektrolytschicht, welche primäre oder sekundäre Aminogruppen umfasst, kann beispielsweise mit Dialdehyden bewirkt werden, wie Glutaraldehyd oder Malondialdehyd. Eine Vernetzung ist auch durch Dicarbonsäuren möglich, die zunächst mit einem Carbodiimid aktiviert werden. Eine anionische Polyelektrolytschicht, welche Carboxylgruppen umfasst, kann beispielsweise durch Di- oder Polyamine vernetzt werden, wie Ethylendiamin oder Diethylentriamin. Eine Vernetzung ist auch unter Verwendung von Hydrazin möglich.

Das erfindungsgemäße Trägermaterial eignet sich für die Adsorption von Biomolekülen. Gemäß einer Ausführungsform der Erfindung ist daher auf dem Trägermaterial ein Biomolekül gebunden. Die Bindung kann dabei reversibel oder irreversibel ausgestaltet sein. Bevorzugt erfolgt die Bindung der Biomoleküle über nicht kovalente Bindungen, wie ionische Bindungen oder Dipol-Dipol-Wechselwirkungen.

Unter einem Biomolekül wird eine Verbindung verstanden, die auf eine biologische Quelle zurückgeht oder mit biotechnologischen Verfahren zugänglich ist. Gemäß einer Ausführungsform weisen die Biomoleküle zumindest eine positiv oder negativ geladene Gruppe auf. Das Molekülgewicht der Biomoleküle liegt bevorzugt im Bereich von 10.000 bis 10.000.000 g/mol. Charakteristisch für Biomoleküle ist ihre leichte Bioabbaubarkeit. Ihr Einsatz stößt daher auf geringe ökologische Bedenken. Nachteilig ist jedoch die vergleichsweise geringe Stabilität der Biomoleküle bzw. der aus diesen hergestellten Materialien.

Bevorzugt sind die Biomoleküle ausgewählt aus der Gruppe von Proteinen, Oligonucleotiden, DNA, RNA, sowie Oligo- und Polysacchariden.

Bevorzugte Proteine sind beispielsweise Enzyme, Glycoproteine, sowie auch kleinere Peptide. Als Enzyme können beispielsweise solche Enzyme verwendet werden, die in einem biotechnologischen Verfahren verwendet werden sollen und daher in geträgerter Form bereitgestellt werden. Ein bevorzugtes Enzym ist beispielsweise Glucoseisomerase.

Weitere geeignete Biomoleküle, welche auf dem erfindungsgemäßen Trägermaterial adsorbiert werden können, sind Polysaccharide, welche geladene Gruppen tragen, wie Chitosane, Alginate, Carrageenan, Chondroitsulfat und modifizierte Zellulose.

Die Erfindung betrifft weiter ein Verfahren zur Herstellung eines Trägermaterials, wie es oben beschrieben worden ist. Bei der Herstellung des Trägermaterials werden schrittweise Schichten aus gegensinnig geladenen Polyelektrolyten auf einen inerten Trägerkern aufgetragen, wobei die erste Schicht des ersten Polyelektrolyts eine gleichsinnige Ladung aufweist, wie der inerte Trägerkern.

Das erfindungsgemäße Verfahren zur Herstellung des oben beschriebenen Trägermaterials umfasst zumindest die folgenden Schritte:
a.) Bereitstellen eines inerten Trägerkerns, welcher eine Oberfläche mit einer positiven oder negativen Gesamtladung aufweist;
b.) Bereitstellen eines ersten Polyelektrolyts, welcher eine zur Oberflächenladung des inerten Trägerkerns gleichsinnige Gesamtladung aufweist;
c.) Beschichten des inerten Trägerkerns mit dem ersten Polyelektrolyt;
d.) Bereitstellen eines zweiten Polyelektrolyts, welcher eine zum ersten Polyelektrolyt gegensinnige Gesamtladung aufweist;
e.) Beschichten des mit dem ersten Polyelektrolyt beschichteten inerten Trägerkerns mit dem zweiten Polyelektrolyt.

In einem ersten Schritt wird der inerte Trägerkern, welcher vorzugsweise aus einem porösen Material aufgebaut ist, in einem geeigneten Lösungsmittel suspendiert. Als Lösungsmittel wird bevorzugt Wasser verwendet. Zur Einstellung der Ionenstärke und des pH-Wertes kann dem Lösungsmittel, insbesondere Wasser, auch ein geeigneter Puffer beigegeben sein. Der pH-Wert ist abhängig vom ersten Polyelektrolyt, der sich beim gewählten pH-Wert im Lösungsmittel lösen sollte. Bevorzugt wird das Lösungsmittel, insbesondere Wasser, auf einen pH-Wert im Bereich von 2 bis 10, besonders bevorzugt 4 bis 8 eingestellt. Die Konzentration des Puffers bzw. von dem Wasser zugegebenen Salzen wird bevorzugt im Bereich von 10 bis 1.000 mmol/l, besonders bevorzugt 50 bis 500 mmol/l gewählt. Der Feststoffgehalt der Suspension des inerten Trägers wird bevorzugt in einem Bereich von 10 bis 500 g/l, besonders bevorzugt 50 bis 200 g/l gewählt. Ggf. kann die Suspension mit Ultraschall behandelt werden, um eine feine Dispergierung des inerten Trägerkerns im Lösungsmittel zu gewährleisten. Die Suspension wird bevorzugt auf eine Temperatur im Bereich von 5 bis 70 °C, besonders bevorzugt 15 bis 50 °C eingestellt. Besonders bevorzugt wird die Herstellung des Trägermaterials bei Raumtemperatur durchgeführt.

Zur Suspension des inerten Trägerkerns wird anschließend der erste Polyelektrolyt zugegeben. Der erste Polyelektrolyt weist eine gleichsinnige Ladung auf wie der inerte Trägerkern. Bevorzugt wird der erste Polyelektrolyt in Form einer Lösung bereitgestellt. Als Lösungsmittel wird bevorzugt Wasser verwendet. Das Lösungsmittel kann einen Puffer zur Einstellung des pH-Wertes enthalten. Die Konzentration des ersten Polyelektrolyts wird bevorzugt im Bereich von 0,1 bis 100 g/l, besonders bevorzugt 0,5 bis 10 g/l gewählt. Die Menge des ersten Polyelektrolyts wird bevorzugt so gewählt, dass ein Überschuss gegenüber der belegbaren Fläche des inerten Trägerkerns vorliegt. Die erforderliche Konzentration kann ggf. durch Vorversuche bestimmt werden, in welchen beispielsweise die Konzentration des nicht adsorbierten ersten Polyelektrolyts im Überstand bestimmt wird. Die Lösung des ersten Polyelektrolyts wird dann zur Suspension des inerten Trägerkerns gegeben. Es ist jedoch auch möglich, den ersten Polyelektrolyt direkt zur Suspension des inerten Trägerkerns zu geben. Der erste Polyelektrolyt wird dann mit dem inerten Trägerkern umgesetzt. Die Dauer der Umsetzung ist von den gewählten Reaktionsparametern abhängig. Bevorzugt wird die Dauer der Umsetzung im Bereich von 1 bis 180 Minuten, besonders bevorzugt 10 bis 30 Minuten gewählt. Während der Umsetzung kann die Suspension bewegt werden, um ein Absinken des inerten Trägerkerns bzw. die Entstehung von Konzentrationsschwankungen im Lösungsmittel zu vermeiden.

Nach der Umsetzung wird der nun mit einer ersten Schicht des ersten Polyelektrolyts versehene inerte Trägerkern vom Lösungsmittel abgetrennt. Dies kann mit üblichen Verfahren erfolgen, beispielsweise Filtrieren oder Dekantieren, ggf. nach vorherigem Zentrifugieren. Der abgetrennte Feststoff kann mit Lösungsmittel, welches ggf. einen Puffer enthalten kann, gewaschen werden.

Der mit dem ersten Polyelektrolyt beschichtete inerte Trägerkern wird erneut in einem Lösungsmittel suspendiert. Der Feststoffgehalt der Suspension wird in einem Bereich gewählt, wie er bereits bei der Suspension des inerten Trägerkerns beschrieben wurde. Als Lösungsmittel wird bevorzugt Wasser verwendet, welches ggf. mit einem Puffer versehen wurde. Der pH der Suspension wird bevorzugt so eingestellt, dass der zweite Polyelektrolyt in gelöster Form vorliegt. Bevorzugt wird der pH-Wert in einem Bereich von 2 bis 10, besonders bevorzugt 4 bis 8 eingestellt.

Der zweite Polyelektolyt wird bevorzugt als Lösung, insbesondere wässrige Lösung, bereitgestellt, die auf einen pH-Wert von vorzugsweise im Bereich von 2 bis 10 eingestellt ist. Die Lösung des zweiten Polyelektrolyts wird dann zur Suspension des mit dem ersten Polyelektrolyt belegten inerten Trägerkerns gegeben. Die Umsetzung wird bei Bedingungen durchgeführt, wie sie bereits bei der Umsetzung des ersten Polyelektrolyts mit dem inerten Trägerkern erläutert wurden.

Der nun auch mit dem zweiten Polyelektrolyt belegte inerte Trägerkern wird vom Lösungsmittel abgetrennt und ggf. gewaschen.

In der gleichen Weise wie für den ersten und zweiten Polyelektrolyt beschrieben, können weitere Schichten eines Polyelektrolyts aufgetragen werden, wobei aufeinander folgende Polyelektrolytschichten jeweils alternierende Vorzeichen ihrer Ladung tragen.

Die Polyelektrolytschichten können nach dem Auftragen noch vernetzt werden. Dazu kann eine, vorzugsweise wässrige, Suspension des beschichteten inerten Trägerkerns mit einem zumindest bifunktionellen Vernetzungsagens versetzt werden. Das Vernetzungsagens wird bevorzugt in Form einer wässrigen Lösung eingesetzt. Die Konzentration des Vernetzungsagens in der Lösung wird bevorzugt im Bereich von 0,05 bis 1,5 Gew.-% gewählt.

Geeignete Vernetzungsagenzien sind beispielsweise Dialdehyde, wie Glutaraldehyd oder Malondialdehyd. Dialdehyde eignen sich insbesondere zur Vernetzung von kationischen Polyelektrolyten, welche Aminogruppen umfassen.

Zur Vernetzung wird der pH-Wert der Suspension bevorzugt in einem Bereich von 5 bis 8,5 eingestellt. Die Reaktionszeit wird bevorzugt im Bereich von 10 bis 60 Minuten gewählt.

Nach dem Vernetzen wird der Feststoff vom Lösungsmittel abgetrennt und ggf. gewaschen.

Ein weiteres geeignetes Vernetzungsmittel sind Carbodiimide. Sie eignen sich insbesondere für Polyelektrolyte, welche Carboxylgruppen umfassen. Ein geeignetes Carbodiimid ist beispielsweise EDAC (1-Ethyl-3-(3-dimethylaminopropyl)carbodiimid. Es wird bevorzugt in Form einer 1 Gew.-% igen Lösung eingesetzt. Zum Vernetzen wird die Suspension des beschichteten Trägerkerns vorzugsweise auf einen pH-Wert im Bereich von 6 bis 8,5 eingestellt. Die Reaktionszeit beträgt bevorzugt 5 bis 60 Minuten. Nach dem Vernetzen wird der Feststoff vom Lösungsmittel abgetrennt und ggf. gewaschen.

Um Biomoleküle auf dem Trägermaterial aufzubringen wird das Trägermaterial zunächst in einem, vorzugsweise wässrigen, Lösungsmittel suspendiert. Das Lösungsmittel enthält vorzugsweise einen Puffer, mit welchem der pH-Wert der Suspension geeignet eingestellt werden kann. Der pH-Wert wird geeignet so eingestellt, dass keine Deaktivierung des Biomoleküls eintritt und eine Adsorption des Biomoleküls an dem Trägermaterial erreicht wird, das Biomolekül also bevorzugt geladene Gruppen aufweist.

Das Biomolekül wird vorzugsweise in Form einer wässrigen Lösung bereitgestellt, welche bevorzugt auf einen geeigneten pH-Wert eingestellt ist. Die Konzentration des Biomoleküls wird bevorzugt im Bereich von 0,1 bis 100 g/l, besonders bevorzugt 0,5 bis 10 g/l gewählt.

Die Zeit für die Adsorption des Biomoleküls am Trägermaterial ist abhängig vom gewählten Biomolekül. Bevorzugt wird die Zeit für die Umsetzung im Bereich von 1 bis 180 Minuten, besonders bevorzugt 10 bis 30 Minuten gewählt.

Nach der Umsetzung kann das Trägermaterial vom Lösungsmittel abgetrennt und ggf. gewaschen werden.

Ein weiterer Gegenstand der Erfindung betrifft die Verwendung des oben beschriebenen Trägermaterials zur Immobilisierung von Biomolekülen.

Die Immobilisierung kann genutzt werden, um störende Biomoleküle aus einem Medium, beispielsweise einem Abwasser, zu entfernen.

Die Immobilisierung kann in der Weise durchgeführt werden, dass die Biomoleküle irreversibel an dem Trägermaterial gebunden werden. Dies ist beispielsweise erwünscht, wenn die Biomoleküle, beispielsweise Enzyme, an einen festen Träger gebunden werden sollen, um anschließend in einer Reaktion eingesetzt werden zu können.

Die Immobilisierung kann gemäß einer Ausführungsform auch reversibel erfolgen. Dies kann beispielsweise erwünscht sein, um ein gewünschtes Biomolekül anzureichern oder beispielsweise aus einem Kulturmedium abzutrennen. Die Abtrennung der gebundenen Biomoleküle kann beispielsweise durch eine Änderung der Salzkonzentration oder des pH-Wertes bewirkt werden. Ferner kann die reversible Immobilisierung der Biomoleküle auch in Form einer Säulenchromatographie erfolgen.

Die Erfindung wird im Weiteren anhand von Beispielen und unter Bezugnahme auf die beigefügten Figuren genauer erläutert. Dabei zeigt:
Fig. 1: eine mikroskopische Aufnahme eines Trägerkerns, welcher mit einem Polystryrolsulfonat beschichtet ist, das mit Rhodamin (PSS-Rho) gelabelt wurde;
Fig. 2: eine Grafik, in welcher die Fluoreszenzintensität für verschiedene Trägerkerne dargestellt ist, welche bei verschiedenem pH mit PSS-Rho beschichtet wurden;
Fig. 3: eine Grafik, in welcher der Anteil an desorbiertem Polyethylenimin dargestellt ist, welcher nach Behandlung beschichteter Trägerkerne in wässrigen Lösungen bei unterschiedlichen Ionenstärken und pH-Werten freigesetzt werden;
Fig. 4: eine Grafik, in welcher der Anteil an adsorbiertem Polyethylenimin und Polystyrolsulfonat für verschieden vorbehandelte Trägerpartikel dargestellt ist;
Fig. 5: eine Grafik, in welcher der prozentuale Anteil an desorbiertem Polystyrolsulfonat dargestellt ist, welches nach Behandlung verschiedener beschichteter Trägerkerne in wässriger Lösung bei einem pH von 9,5 freigesetzt wird.

### Verwendete Messmethoden:

### BET-Oberfläche/Porenvolumen nach BJH und BET:

Die Oberfläche und das Porenvolumen wurden mit einem vollautomatischen Stickstoffporosimeter der Firma Mikromeritics, Typ ASAP 2010 bestimmt.

Die Probe wird im Hochvakuum auf die Temperatur von flüssigem Stickstoff abgekühlt. Anschließend wird kontinuierlich Stickstoff in die Probenkammer dosiert. Durch die Erfassung der adsorbierten Gasmenge als Funktion des Druckes wird bei konstanter Temperatur eine Adsorptionsisotherme ermittelt. Nach einem Druckausgleich wird das Analysengas schrittweise entfernt und eine Desorptionsisotherme aufgenommen.

Zur Ermittlung der spezifischen Oberfläche und der Porosität nach der BET-Theorie werden die Daten gemäß DIN 66131 ausgewertet.

Das Porenvolumen wird ferner aus den Messdaten unter Anwendung der BJH-Methode ermittelt (E.P. Barret, L.G. Joiner, P.P. Haienda, J. Am. Chem. Soc. 73 (1951, 373). Bei diesem Verfahren werden auch Effekte der Kapillarkondensation berücksichtigt. Porenvolumina bestimmter Porengrößenbereiche werden durch Aufsummieren inkrementeller Porenvolumina bestimmt, die aus der Auswertung der Adsorptionsisotherme nach BJH erhalten werden. Das Gesamtporenvolumen nach der BJH-Methode bezieht sich auf Poren mit einem Durchmesser von 1,7 bis 300 nm.

### Teilchengrößenbestimmung mittels dynamischer Lichtstreuung (Malvern)

Die Messungen werden mit einem Gerät "Mastersizer" der Firma Malvern Instruments Ltd., UK, entsprechend den Angaben des Herstellers durchgeführt. Die Messungen werden mit der vorgesehenen Probenkammer ("dry powder feeder") in Luft durchgeführt und die auf das Probenvolumen bezogenen Werte ermittelt.

### Wassergehalt:

Der Wassergehalt der Produkte bei 105°C wird unter Verwendung der Methode DIN/ISO-787/2 ermittelt.

### Bestimmung des Zetapotentials:

Das Zetapotential wurde anhand der elektrophoretischen Mobilität im elektrischen Feld bei einem pH-Wert von 5,6 in 50 mmolarem Acetatpuffer mit dem Gerät "Zetasizer" der Firma Malvern Instruments Ltd. UK entsprechend den Angaben des Herstellers durchgeführt. Es wurde ein Feststoffgehalt(Trägergehalt) von 0,1 Gew.-% eingestellt.

### Bestimmung des Molekülgewichts von Polyelektrolyten

Das Molekülgewicht der Polyelektrolyte wird durch Größenausschlußchromatographie und Vergleich mit Polyacrylat-Standards ermittelt.

### Elementaranalyse:

Diese Analyse beruht auf dem Totalaufschluss der Tonmaterialien bzw. des entsprechenden Produktes. Nach dem Auflösen der Feststoffe werden die Einzelkomponenten mit herkömmlichen spezifischen Analysemethoden, wie z.B. ICP analysiert und quantifiziert.

### Ionenaustauschkapazität:

Zur Bestimmung der Kationenaustauschkapazität wird das zu untersuchende Tonmaterial über einen Zeitraum von 2 Stunden bei 105°C getrocknet. Danach wird das getrocknete Tonmaterial mit einem Überschuss an wässriger 2N NH₄Cl-Lösung 1 Stunde unter Rückfluss zur Reaktion gebracht. Nach einer Standzeit von 16 Stunden bei Raumtemperatur wird filtriert, worauf der Filterkuchen gewaschen, getrocknet und vermahlen wird und der NH₄-Gehalt im Tonmaterial durch Stickstoffbestimmung (CHN-Analysator "Vario EL III" der Firma Elementar in Hanau) nach den Herstellerangaben ermittelt. Der Anteil und die Art der ausgetauschten Metallionen wird im Filtrat durch ICP-Spektroskopie bestimmt.

### Bestimmung des Trockensiebrückstandes

Etwa 50 g des zu untersuchenden lufttrockenen Minerals werden auf einem Sieb der Maschenweite 45 µm eingewogen. Das Sieb wird an einen Staubsauger angeschlossen, der über ein unter dem Siebboden kreisenden Saugschlitz alle Anteile, die feiner als das Sieb sind, durch das Sieb heraussaugt. Das Sieb wird mit einem Plastikdeckel abgedeckt und der Staubsauger eingeschaltet. Nach 5 Minuten wird der Staubsauger abgeschaltet und die Menge der auf dem Sieb verbliebenen gröberen Anteile durch Differenzwägung ermittelt.

### Bestimmung des Nasssiebrückstandes

Es wird zunächst eine 5 %-ige Suspension hergestellt, indem eine entsprechende Menge des zu untersuchenden Tonmaterials bei ca. 930 UpM ca. 5 Minuten in Wasser eingerührt wird. Die Suspension wird für weitere 15 Minuten bei ca. 1865 UpM gerührt und die Suspension dann durch ein Sieb der gewünschten Maschenweite gegossen. Der Rückstand wird so lange mit Leitungswasser gewaschen, bis das Waschwasser klar abläuft. Das Sieb mit dem Rückstand wird dann für 5 Minuten in ein Ultraschallbad gesetzt, um restliche Feinanteile zu entfernen. Der verbliebene Rückstand wird kurz mit Leitungswasser gewaschen und die Ultraschallbehandlung ggf. wiederholt, bis während der Ultraschallbehandlung keine Feinstoffe mehr in das Wasser übertreten. Das Sieb wird dann bis zur Gewichtskonstanz getrocknet. Zum Auswiegen wird der auf dem Sieb verbliebene Rückstand in eine gewogene Porzellanschale überführt.

### Bestimmung des Methylenblauwertes

Der Methylenblauwert ist ein Maß für die innere Oberfläche der Tonmaterialien.
a) Herstellung einer Tetranatriumdiphosphat-Lösung
   5,41 g Tetranatriumdiphosphat werden auf 0,001 g genau in einen 1000 ml Messkolben eingewogen und unter Schütteln bis zur Eichmarke mit dest. Wasser aufgefüllt.
b) Herstellung einer 0,5 %-igen Methylenblaulösung
   In einem 2000 ml Becherglas werden 125 g Methylenblau in ca. 1500 ml dest. Wasser gelöst. Die Lösung wird abdekantiert und auf 25 1 mit dest. Wasser aufgefüllt.
   0,5 g feuchter Testbentonit mit bekannter innerer Oberfläche werden in einem Erlenmeyerkolben auf 0,001 g genau eingewogen. Es werden 50 ml Tetranatriumdiphosphatlösung zugegeben und die Mischung 5 Minuten zum Sieden erhitzt. Nach dem Abkühlen auf Raumtemperatur werden 10 ml 0,5 molare H₂SO₄ zugegeben und 80 bis 95 % des zu erwartenden Endverbrauchs an Methylenblaulösung zugegeben. Mit dem Glasstab wird ein Tropfen der Suspension aufgenommen und auf ein Filterpapier gegeben. Es bildet sich ein blau-schwarzer Fleck mit einem farblosen Hof. Es wird nun in Portionen von 1 ml weitere Methylenblaulösung zugegeben und die Tüpfelprobe wiederholt. Die Zugabe erfolgt solange, bis sich der Hof leicht hellblau färbt, also die zugegebene Methylenblaumenge nicht mehr vom Testbentonit absorbiert wird.
c) Prüfung von Tonmaterialien
   Die Prüfung des Tonmaterials wird in der gleichen Weise durchgeführt wie für den Testbentonit. Aus der verbrauchten Menge an Methylenblaulösung lässt sich die innere Oberfläche des Tonmaterials berechnen.

### Bestimmung des pH-Wertes einer Bentonitprobe

2 g der Probe werden in 98 ml destilliertem Wasser dispergiert. Danach wird mit einer kalibrierten Glaselektrode der pH-Wert bestimmt.

### Allgemeine Methoden

### 1. Herstellung fluoreszenzgelabelter Polyelektrolyte

Die Polymere wurden über zwei verschiedene Methoden mit Fluoreszenzfarbstoffen gelabelt. Sofern das Polymer kopplungsfähige Aminogruppen aufweist, wird ein Teil der Gruppen mit Rhodamin- bzw. Fluoreszeinisothiocyanat umgesetzt. Sofern das Polymer keine kopplungsfähigen Aminogruppen aufweist, werden die Monomere Acrylrhodamin bzw. Acrylfluoreszein durch Copolymerisation während der Herstellung der Polymere eingeführt. Nach der Reaktion werden die entstandenen Reaktionsgemische durch exzessive Dialyse gegen Salz und anschließend Wasser gereinigt. Im letzten Schritt werden die Substanzen getrocknet und der Labelgrad mit UV-Vis-Spektroskopie bestimmt.

### 1.1. Herstellung von mit Fluoreszein gelabelten Poly(ethyleniminen) (PEI-FLU)

Es wurden 2 verschiedene Polyethylenimine mit Molekülgewichten von 750.000 (A = 920 mg) und 25 000 (B = 460 mg) g/mol eingesetzt

Die Zugabe von dem Labelfarbstoff Fluoreszeinisothiocyanat wurde auf einen theoretischen Labelgrad von Farbstoff:Monomer = 1:75 berechnet

Das PEI wurde in 10 ml (A) bzw. 5 mL (B) Wasser gelöst und die Lösung durch Zugabe von 2M HCl auf pH 8,4 eingestellt. 60 mg des Farbstoffes wurden in 4ml DMF gelöst und langsam unter Rühren zu den PEI Lösungen (3.3 mL zu A und 1.7 mL zu B) zugegeben. Nach 1 h Reaktionszeit bei Raumtemperatur wurde der pH-Wert mit NaOH auf 8,4 nachgestellt. Dann wurde über Nacht bei Raumtemperatur weiter gerührt.

Nach weiteren 4h Reaktion bei erhöhter Temperatur im Wasserbad bei 45°C wurde der pH-Wert mit HCl auf pH 7 neutralisiert. Dann wurden die Lösungen mit 15 kDA Dialyseschlauch bei täglichem Wechsel des Dialysemediums dialysiert bis die äußere Lösung im UV/Vis Spektrometer frei von Farbstoff war (6 Tage). Anschließend wurden die Lösungen in Plastikgefäße filtriert (Papierfilter), der pH-Wert mit Salzsäure auf 4,5 eingestellt und die Lösungen eingefroren.

Von den Lösungen wurde die Konzentration und der Labelgrad folgendermaßen bestimmt (Doppelbestimmung). Von jedem Ansatz werden jeweils 2 x 1 ml abgenommen und in vorher exakt ausgewogene Eppendorfröhrchen gefüllt. Die Proben werden eingefroren und dann dass Wasser durch Gefriergetrocknen entfernt. Die Masse des zurückbleibenden Polymers wird durch Differenzwägung bestimmt. Nach dem Auswiegen werden die Polymere in 0,05M TRIS- Puffer pH 7,0 gelöst, verdünnt und die Extinktion im Maximum bei 489 nm am UV-Vis Spektrometer gemessen.

Die Auswertung ergab die in der folgenden Tabelle aufgeführten Masse bzw. Konzentration des Polymers in der Lösung. Über das Lambert-Beersche Gesetz wurde aus den UV/Vis Spektren die Konzentration des Fluoreszeins ausgerechnet, wobei ein Extinktionskoeffizient aus der Literatur von 80 000 l/g · mol zugrunde gelegt wurde. Daraus ergaben sich die in Tabelle 1 angegebenen Labelgrade.

**Tabelle 1: Daten zur Einführung eines Labels in verschiedene Polyethylenimine**

| Nr. | c (g/l) | Polymer | Extinktion pH 7,0 | C_{Farbstoff} (mol/l x10⁻⁴) | C_{polymer} mol/l | Mol Farb-stoff/ g Polymer mol/l x10⁻⁴ | Labelgrad |
|---|---|---|---|---|---|---|---|
| FITC-A1 | 1,42 | PEI₇₅₀₀₀₀ | 16,2 | 2,025 | 0,018 | 1,426 | 1 : 88,9 |
| FITC-A2 | 1,64 | PEI₇₅₀₀₀₀ | 16,3 | 2,037 | 0,0208 | 1,242 | 1 : 102 |
| Labeleffizienz: 79,2% Extinktion bei: 489,0nm | | | | | | | |
| FITC-B1 | 1,52 | PEI₂₅₀₀₀ | 16,9 | 2,11 | 0,019 | 1,38 | 1 : 90 |
| FITC-B2 | 1,34 | PEI₂₅₀₀₀ | 16,0 | 2 | 0,017 | 1,49 | 1 : 85 |
| Labeleffizienz: 86,4% Extinktion bei: 489,0nm | | | | | | | |

### 1.2. Herstellung von mit Rhodamin gelabeltem Polystyrolsulfonatnatriumsalz (PSS-Rho)

500 mg Natriumstyrolsulfonat wurden in 2.5 mL 0.2 M NaCl Lösung aufgelöst. Das molare Verhältnis von Monomer zu Farbstoff wurde mit 50 : 1 eingestellt. Dazu wurden 33 mg Methacryloxyethylthiocarbamoyl-rhodamin B (Polyscience) in 1 mL DMF aufgelöst und zu der Styrolsulfonatlösung gegeben. Dazu wurde im Kalten (10°C) 250 µL einer 3 %-igen (m/m) Kaliumperoxodisulfatlösung in Wasser gegeben und durchmischt. Das Reaktionsgefäß wurde luftdicht mit einem Septum verschlossen und über zwei Kanülen 20 min mit 99.999 % Stickstoff gespült. Dann wurde das Reaktionsgemisch auf 55°C erwärmt und über Nacht unter Rühren bei der Temperatur belassen. Zum Schluss wurde die Temperatur auf 75 °C für weitere zwei Stunden gesteigert.

Die Reaktionslösung wurde in 15 000 kD Dialyseschlauch gefüllt und eine Woche gegen destilliertes Wasser mit täglichem Wechsel des Mediums dialysiert. Die Lösung wurde filtriert und anschließend gefriergetrocknet. Die Analyse des Labelgrades erfolgte wie beim PEI-Flu beschrieben, jedoch wurde die Absorption bei 548 nm gemessen und ein Extinktionskoeffizient von 100 000 l/g · mol für das Rhodamin verwendet.

### 2. Herstellung der inerten Trägerkerne

Für die Untersuchungen wurden verschiedene Träger hergestellt, die jeweils eine anionische Gesamtladung bzw. ein negatives Zetapotential aufweisen.

### 2.1. Herstellung des Trägermaterials 1 aus sauer aktiviertem Bentonit

Für die Herstellung des Trägermaterials 1 wurden sauer aktivierte Bentonite verwendet. Die Eigenschaften der Ausgangsmaterialien sind in Tabelle 2 zusammengefasst. Die Ausgangsmaterialien sind von der Süd-Chemie AG, München, DE erhältlich.

**Tabelle 2: physikalische Eigenschaften der Ausgangsmaterialien**

| | Bleicherde 1 (Pulver) | Bleicherde 2 (Pulver) | Ca/Na-Bentonit | Granulat |
|---|---|---|---|---|
| BET-Oberfläche m²/g | 361,75 | 277,02 | 63,7 | 270 |
| Kumuliertes Porenvolumen nach BJH für Poren mit Durchmessern von 1,7 -300 nm [cm³/g] | 0,4535 | 0,316 | 0,0743 | 0,3645 |
| Mittlerer Porendruchmes-ser nach BJH [nm] | 4,9 | 4,8 | 7,4 | 5,7 |
| Nasssiebrückstand (%) | | | | |
| 63 µm | 10,1 | 21,5 | 15,7 | 99 |
| 45 µm | 15,7 | 30,0 | 41,8 | 99d |
| Trockensiebrückstand (%) | | | | |
| 63 µm | n. b. | n. b. | n. b. | 99 |
| 45 µm | n. b. | n. b. | n. b. | 100 |
| pH-Wert | 3,04 | 3,74 | 6,54 | 4,19 |
| Schüttgewicht (g/l) | - | - | - | 555 |

60 kg Bleicherde 1, 32 kg Bleicherde 2 und 8 kg Ca/Na-Bentonit werden in einem Mischer homogenisiert. Danach werden 35 kg Wasser zugesetzt und die Mischung für weitere 30 Minuten geknetet. Die plastische Masse wird in eine Ringscheibenpresse (Flachmatrizenpresse) der Firma Amandus Kahl GmbH & Co KG, Reinbek, DE mit einem Lochdurchmesser von 3 mm unter stark kompaktierenden Bedingungen zu Strangpresslingen verformt. Ein mitlaufendes Schneidwerkzeug kürzt die Presslinge auf eine Länge von 2 bis 3 mm. Die feuchten Extrudate werden in einem Trommeltrockner getrocknet und auf eine Teilchengröße von 0,5 bis 2 mm abgesiebt. Überkorn wird gebrochen und auf das Sieb zurückgeführt. Das erhaltene Granulat wird für eine Stunde bei 600 °C calciniert. Die Eigenschaften des Granulats sind ebenfalls in Tabelle 1 aufgeführt.

Die Granulate weisen einerseits eine hohe mechanische Stabilität auf und besitzen andererseits noch eine hohe Porosität.

### 2.2. Herstellung der Trägermaterialien 2, 3 und 4 von Trägern (Granulaten) aus Bentonit und Saponit

Die Trägermaterialien 2, 3 und 4 wurden jeweils aus verschiedenen Bentoniten sowie einem Saponit hergestellt. Die Eigenschaften der als Ausgangsmaterial verwendeten Tonmineralien sind in den Tabellen 3a bis c zusammengefasst.

**Tabelle 3a: physikalische Eigenschaften des Bentonits 1**

| | Bentonit 1 |
|---|---|
| Montmorillonitgehalt, bestimmt nach der Methylenblau-methode [%] | 75 |
| Kationenaustauschkapazität [meq/100 g] | 76 |
| BET-Oberfläche [m²/g] | 80 |
| Kumulatives Porenvolumen nach der BJH-Methode für Poren mit Durchmessern von 1,7 bis 300 nm, [cm³/g] | 0,19 |
| Durchschnittsporendurchmesser [4V/A] nach BJH | 11,1 |
| Trockensiebrückstand auf 45 µm (Gew.-%) | 13,2 |
| Nasssiebrückstand auf 45 µm (Gew.-%) | 14,7 |
| Nasssiebrückstand auf 25 µm (Gew.-%) | 21,2 |

**Tabelle 3b: physikalische Eigenschaften des Bentonits 2**

| | Bentonit 2 |
|---|---|
| Montmorillonitgehalt, bestimmt nach der Methylen-blaumethode [%] | 100 |
| Kationenaustauschkapazität [meq/100 g] | 105 |
| Trockensiebrückstand auf 45 µm (Gew.-%) | 11 |
| Nasssiebrückstand auf: | |
| 45 µm (Gew.-%) | 0,9 |
| 25 µm (Gew.-%) | 7,3 |

**Tabelle 3c: physikalische Eigenschaften des Saponits 1**

| | |
|---|---|
| BET- Oberfläche [m²/g] | 125,3 |
| Gesamtkationenaustauschkapazität [meq/100 g] | 46 |
| Begleitminerale | 2 - 3 % Quarz |
| | 2 - 3 % Feldspat |
| | 0,5 - 1 % Calcit |
| Silikatanalyse [Gew.-%] | |
| Al₂O₃ | 6,6 |
| Fe₂O₃ | 1,9 |
| CaO | 1,1 |
| MgO | 26,0 |
| Na₂O | 0,32 |
| K₂O | 1,4 |
| TiO₂ | 0,25 |
| SiO₂ | 52,0 |
| Verhältnis SiO₂/MgO | 2,00 |
| Glühverlust | 9,5 |

In einem Eirich Intensivmischer R02E (Firma Eirich, Hartheim, DE) wurden jeweils 1500 g des Tonminerals vorgelegt und über einen Trichter 400 ml Wasser als Agglomerationsmittel zudosiert. Dabei wurde die niedrige Einstellung für die Umdrehungsgeschwindigkeit des Tellers sowie die maximale Umdrehungsgeschwindigkeit für den Wirbler gewählt. Die nassen Granulate wurden zunächst bei 70 °C getrocknet und dann während einer Stunde einer thermischen Behandlung bei der angegebenen Temperatur unterworfen. Die Granulate wurden dann in einem Größenbereich von 0,1 bis 1,5 mm abgesiebt. Die physikalischen Eigenschaften der Granulate sind in Tabelle 4 zusammengefasst.

**Tabelle 4: physikalische Eigenschaften der Träger (Granulate)**

| | Träger 2 (aus Bentonit 1) | Träger 3 (aus Bentonit 2) | Träger 4 (aus Saponit 1) |
|---|---|---|---|
| Schüttgewicht [g/l] | 780 + 100 | 1060 + 150 | 835 + 100 |
| pH (5 Gew.-% in Wasser) | 8,5 | 9,3 | 8,3 |
| BET-Oberfläche [m²/g] | 58 | 67 | 45 |
| Wassergehalt (Gew.-%) | < 3 | < 4 | < 3 |

### 2.3. Herstellung von Trägern auf der Basis von Fällungskieselsäure (Trägermaterialien 5 bis 9)

Zum Vergleich wurden Granulate auf Basis von Fällungskieselsäure hergestellt. Dazu wurde zum Einen reine Fällungskieselsäure (Sipernat^{®} 22, Evonik Degussa, Hanau, DE) mit Wasser granuliert. Zum Anderen wurden der Kieselsäure verschiedene Bentonite als Bindemittel zugegeben. Ferner wurde bei der Herstellung von einigen der Granulate Wasserglas als weiteres Bindemittel zugesetzt. Die Granulierung wurde in analoger Weise wie unter 2.2. beschrieben durchgeführt. Nach dem Trocknen wurden die Granulate noch jeweils für eine Stunde bei 600 °C gesintert. Die für die Herstellung der Granulate verwendeten Formulierungen sind in Tabelle 5 zusammengefasst.

**Tabelle 5: Formulierungen zur Herstellung von Granulaten auf der Basis von Fällungskieselsäure**

| | Träger 5 | Träger 6 | Träger 7 | Träger 8 | Träger 9 |
|---|---|---|---|---|---|
| Einwaage Sipernat^{®} 22 [g] | 300 | 320 | 360 | 200 | 200 |
| Einwaage Bleicherde Tonsil^{®} Optimum 210 FF [g] | - | - | - | - | 200 |
| Einwaage Bentonit Laundrosil^{®} DGA Pul-ver [g] | - | 80 | 40 | 200 | - |
| Wasser [g] | 506 | 712 | - | 504 | 480 |
| Verdünntes Wasserglas [g] * | - | - | 830 | - | - |

| | | | | | |
|---|---|---|---|---|---|
| *: ein Wasserglas mit einem Modul SiO₂ : NaO von 3,2, das einen Feststoffgehalt von 36 Gew.-% aufwies, wurde im Verhältnis 1 : 2 mit Leitungswasser verdünnt. | | | | | |

Die Eigenschaften der erhaltenen Granulate sind in Tabelle 6 zusammengefasst.

**Tabelle 6: physikalische Eigenschaften der Granulate**

| | Träger 5 | Träger 6 | Träger 7 | Träger 8 | Träger 9 |
|---|---|---|---|---|---|
| Schüttgewicht [g/l] | 343 | 282 | 402 | 364 | 465 |
| pH (2 Gew.-% in Wasser) | 6,1 | 7,6 | 11,4 | 8,8 | 4,8 |
| Wassergehalt (Gew.-%) | 2,0 | 5,0 | 1,5 | 2,4 | 1,3 |
| BET-Oberfläche (m²/g) | 157 | 131 | 39 | 85 | 181 |
| Kumulatives Porenvolumen nach BJH für Poren von 1,7 - 300 nm (cm³/g) | 1,12 | 0,97 | 0,33 | 0,61 | 0,73 |
| Durchschnitts-porendurchmesser nach BJH (nm) | 28,6 | 28,1 | 38,2 | 27,6 | 15,1 |

### 2.4. Bestimmung der Formbeständigkeit der Granulate in Wasser bzw. wässrigen Pufferlösungen

Die unter 2.1 bis 2.3 erhaltenen Granulate wurden jeweils 7 Tage in Wasser sowie verschiedenen Puffern gelagert. Dazu wurden jeweils 0,5 g des Granulats in 20 ml Flüssigkeit gegeben.

Es wurden die folgenden jeweils 50 mmolaren Puffer verwendet:

| Puffer | pH-Wert |
|---|---|
| Citrat | 3 |
| Na-Acetat | 5 |
| HEPES | 7 |
| TRIS | 9 |

Die Granulate zeigten keine Veränderungen. Es war kein Zerfall erkennbar, der durch die Bildung von Trübungen oder eines feinen Bodensatzes angezeigt würde.

### 2.5. Bestimmung des Zetapotentials

Das Zetapotential der Trägerkerne wurde unter Verwendung der Methode der Bestimmung der Wanderungsgeschwindigkeit im elektrischen Feld bestimmt. Hierzu wurde ein Zetasizer Nano Series der Fa. Malvern Instruments Ltd. (Malvern, Worcestershire, WR14 1XZ, England) eingesetzt. Die Messeinstellungen sind wie folgt:

| | |
|---|---|
| Zugrundegelegtes Messmodel (General Options): | Smoluchowski |
| Zugrundegelegtes Auswerte-modell: | General Purpose |
| Messtemperatur: | 25 °C |

Für die Messungen des Zetapotential wurden die wie in 2.1 bis 2.3 beschrieben erhaltenen Granulate auf eine mittlere Teilchengrößen von 8 ± 5 µm vermahlen. Die so erhaltenen Pulver wurden in einer Konzentrationen von 0,1 Gew.-% in Acetat-Puffer (50 mmol; pH 5,6 ± 0,25) dispergiert. Dazu wurde die Dispersion für 5 Minuten im Ultraschallbad behandelt. Anschließend wurde der pH-Wert der Dispersion mit einer pH-Elektrode bestimmt und unmittelbar daran anschließend das Zetapotential gemessen. Der für die Messungen eingestellte pH entspricht dem pH-Wert, wie er bei der weiter unten beschriebenen Beschichtung der Trägerkerne mit einem Polyelektrolyt eingestellt wird. Die Messungen wurden mehrfach durchgeführt und anschließend der Mittelwert berechnet.

Die Ergebnisse der Messungen sind in Tabelle 7 zusammengefasst

**Tabelle 7: Zetapotentiale verschiedener Trägerkerne (0,1 Gew.-% in 50 mmol Acetatpuffer)**

| Träger | pH-Wert der 0,1 %i-gen Dispersion | Zetapotential [mV] |
|---|---|---|
| Träger 1 | 5,67 | -18,7 |
| Träger 2 | 5,68 | -26,3 |
| Träger 3 | 5,68 | -25,2 |
| Träger 4 | 5,75 | -22,5 |
| Träger 5 | 5,66 | -10,3 |
| Träger 6 | 5,68 | -23,5 |
| Träger 7 | 5,68 | -30,3 |
| Träger 8 | 5,68 | -25,1 |
| Träger 9 | 5,63 | -25,3 |

### 3. Beschichtung der Trägerkerne

Zur Herstellung einer Beschichtungslösung wurde zunächst eine wässrige Lösung von Natriumchlorid und einem Puffer zur Stabilisierung des pH-Wertes hergestellt. Die Konzentrationen sind bei den jeweiligen Beispielen angegeben. In dieser Lösung wurde der betreffende Polyelektrolyt in einer Menge gelöst, dass eine Konzentration von etwa 1 g/l eingestellt wurde.

Zu dieser Beschichtungslösung wurde jeweils das Trägermaterial in der bei den Beispielen angegebenen Menge gegeben und die Suspension am Rotator SB3 mit 10rpm für 30 Minuten inkubiert. Anschließend wurde die Suspension stehen gelassen, so dass sich das Trägermaterial absetzen konnte. Der klare Überstand wurde mit einer Pipette abgenommen. Der feste Rückstand wurde dreimal in bidestilliertem Wasser resuspendiert und die wässrige Phase erneut nach Absetzen lassen des Trägermaterials abgenommen. Anschließend wurde gegebenenfalls für die Adsorption einer weiteren Polyelektrolytschicht der feste gewaschene Rückstand in einer entsprechenden Beschichtungslösung resuspendiert und für 30 Minuten bei Raumtemperatur inkubiert. Das Trägermaterial wurde anschließend in der oben geschilderten Weise abgetrennt und gewaschen.

### Beispiel 1: Adsorption von negativ geladenen Polyelektrolyten auf negativ geladenen porösen Silicatmaterialien

Für die Beschichtung wurden Beschichtungslösungen von PSS-Rho hergestellt. Dazu wurde ein Natriumacetatpuffer (20 mM) verwendet sowie eine NaCl-Konzentration von 0,2 M. Die Beschichtungslösungen wurden auf einen pH-Wert von 4,0 bzw. 5,6 eingestellt.

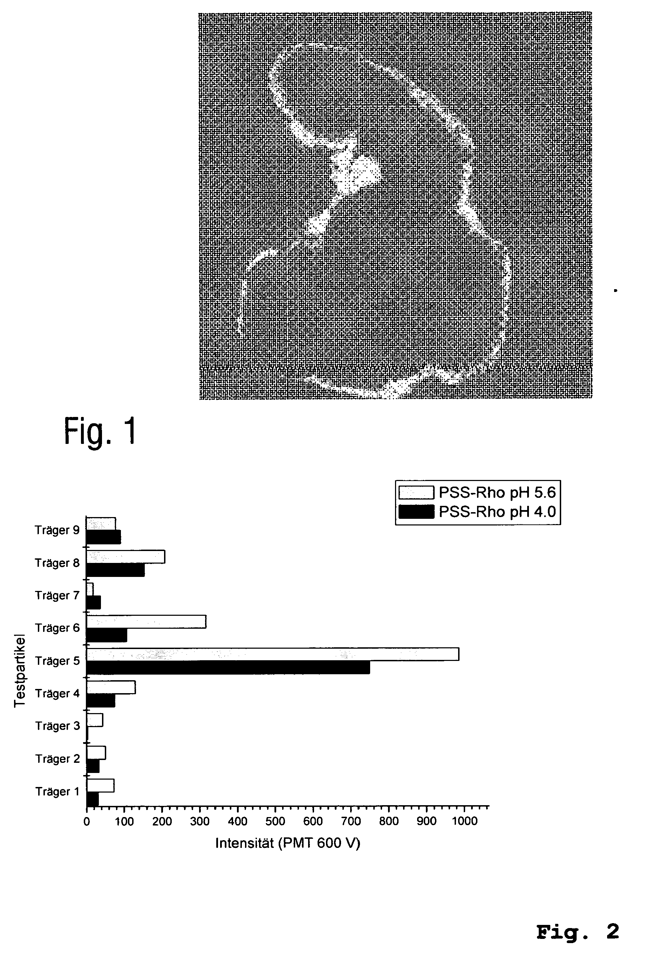

Die Trägerkerne wurden in der im Abschnitt 3 beschriebenen Weise mit den Beschichtungslösungen behandelt. Die erhaltenen beschichteten Trägermaterialien wurden mit konfokaler Laser Scanning Mikroskopie (CLSM) untersucht. Es konnte eine deutliche Fluoreszenz des PSS-Rhodamins auf den Trägermaterialien festgestellt werden. In Fig. 1 ist eine entsprechende konfokale Aufnahme eines Partikels dargestellt. Die Ansicht entspricht einem Schnitt durch eine Ebene des Partikels. Die hellen Bereiche entsprechen dabei der fluoreszierenden Schicht des PSS-Rho. Wie aus der Aufnahme zu erkennen ist, dringt das PSS nicht sehr tief in die poröse Struktur ein. Mit Hilfe der CLSM-Aufnahmen wurde die Fluoreszenzintensität der adsorbierten Schicht vermessen. Die Intensitäten sind in Figur 2 graphisch wiedergegeben. Die ermittelten Intensitäten sind in Tabelle 8 zusammengefasst.

**Tabelle 8: Fluoreszenzintensitäten des adsorbierten PSS-Rhodamin für verschiedene Trägerkerne**

| Träger | Intensität pH der Beschich-tungslösung: 4 | Intensität pH der Beschich-tungslösung: 5,6 |
|---|---|---|
| Träger 1 | 30,5 | 73,2 |
| Träger 2 | 32,8 | 50,5 |
| Träger 3 | 3,1 | 43,6 |
| Träger 4 | 75,4 | 129,7 |
| Träger 5 | 748,6 | 986,2 |
| Träger 6 | 107,8 | 317,1 |
| Träger 7 | 37,7 | 18,9 |
| Träger 8 | 153,9 | 208,5 |
| Träger 9 | 91,7 | 78,8 |

Bei den verschiedenen Testmaterialien wurden deutliche Unterschiede der Menge an adsorbiertem PSS-Rho gefunden. Mit Ausnahme von Träger 7 wurde bei höherem pH-Wert eine höhere Menge an PSS-Rho abgeschieden. Dieses Ergebnis überrascht, da die Partikel bei höherem pH eine stärker negative Ladung aufweisen sollten. Die Adsorption des Polymers auf dem gleichsinnig geladenen Trägermaterial muss daher auf Wechselwirkungskräften beruhen, welche die Abstoßung zwischen gleichsinnig geladenen Gruppen auf der Oberfläche des Trägermaterials sowie am Polyelektrolyt in ihrer Intensität übertreffen.

### Beispiel 2: Adsorption von Polyethylenimin an blankem sowie beschichteten Trägerkernen

Es wurde zunächst eine Beschichtungslösung von mit Fluoreszein gelabeltem Polyethylenimin (PEI-Fluo) hergestellt (Mw 25.000, 1 g/l, 50 mM Acetatpuffer, pH 5,6). Zur Lösung wurden jeweils die in Tabelle 9 aufgeführten inerten Trägerkerne gegeben und jeweils 30 Minuten bei Raumtemperatur inkubiert. Die Menge an adsorbiertem Polyethylenimin wurde durch Messung der Konzentration an PEI-Fluo im Überstand mittels UV/Vis im Vergleich zu Standardlösungen ermittelt. Die ermittelten Werte sind ebenfalls in Tabelle 9 aufgeführt. Weiterhin wurde die Fluoreszentintensität des adsorbierten Polyethylenimin im konfokalen Mikroskop quantitativ analysiert. Die Werte sind ebenfalls in Tabelle 9 aufgeführt. Ferner wurde die Eindringtiefe des Polyelektrolyts aus den mikroskopischen Aufnahmen bestimmt. Die Versuche wurden einmal mit dem unbeschichteten Material des Trägerkerns durchgeführt. In einer zweiten Versuchsreihe wurde das in Beispiel 1 erhaltene, mit PSS-Rho beschichtete Material als Träger verwendet.

**Tabelle 9: Menge an adsorbiertem Polyethylenimin pro Partikel (in Gew.-%) aus Konzentrationsmessungen, Fluoreszenzintensitäten des PEI-Flu aus konfokaler Laser Scanning Mikroskopie sowie Eindringtiefe des Polyethylenimins**

| Trägerkern | % PEI-Fluo mit Vorbeschichtung | Konf. Intens. (650V) | Eindringtiefe in µm | % PEI-Flu ohne Vorbeschichtung | Konf. Intens. (650V) | Eindringtiefe in µm |
|---|---|---|---|---|---|---|
| Träger 1 | 0.66 | 477 | 25 | 0.57 | 165 | 35 |
| Träger 3 | <0.1 | 196 | 20-40 | <0.1 | 73 | 50 |
| Träger 6 | 0.93 | 480 | 20-40 | 0.98 | 225 | 50 |
| Träger 7 | 0.59 | 215 | 40 | 0.27 | 110 | 30-40 |
| Träger 8 | 0.45 | 420 | 40-45 | 0.2 | 500 | 50 |

Die Daten aus Tabelle 9 zeigen, dass die Menge an adsorbiertem Polykation bei einer PSS-Grundierung des Trägermaterials deutlich höher ist als beim unbeschichteten Material. Dadurch kann die Trenneffizienz des Trägers deutlich erhöht werden. Die in den mikroskopischen Aufnahmen ermittelten Intensitäten zeigen zum Teil deutliche Unterschiede in der Menge an adsorbiertem Polyethylenimin. Dies wird offensichtlich durch eine verminderte Eindringtiefe des Polyelektrolyts in die poröse Trägeroberfläche hervorgerufen. Offensichtlich verstopft das adsorbierte PSS bisher kleine Poren. Für die Trenneffizienz spielen die für das Polyethylenimin nicht mehr zugänglichen Poren jedoch keine Rolle, da Proteine im allgemeinen Molekulargewichte über 25.000 aufweisen und damit nicht in kleine Poren eindringen können.

### Beispiel 3: Stabilität von auf inerten Trägerkernen adsorbierten Polyethyleniminschichten

Die in Beispiel 2 erhaltenen Trägermaterialien wurden in Natriumchloridlösungen suspendiert, welche eine Konzentration von 0,2, 0,5, 1 sowie 2 M aufwiesen und über 1 Stunde bei Raumtemperatur inkubiert. Die Suspension wurde zentrifugiert und die Menge an desorbiertem PEI-Fluo im Überstand bei pH 5,6 im UV/Vis-Spektrometer vermessen. Die ermittelte Adsorption lag für alle Proben unterhalb der Nachweisgrenze des UV-Spektrometers, d.h. dass unter den gewählten Bedingungen weniger als 0,3 % der gesamten PEI-Menge freigesetzt wird. Damit kann gezeigt werden, dass die erfindungsgemäßen Trägermaterialien auch bei hohen Ionenstärken stabil sind.

In einer zweiten Versuchsreihe wurden die in Beispiel 2 hergestellten Trägermaterialien in einem 50 mM Acetatpuffer bei pH 4 bzw. pH 5 suspendiert und für eine halbe Stunde bei Raumtemperatur inkubiert. Anschließend wurde die Menge an desorbiertem PEI-Flu wie oben erläutert durch UV/Vis-Spektrometrie ermittelt. Es konnte kein desorbiertes PEI-Flu nachgewiesen werden. Auch bei einer Wiederholung der Versuchsreihe mit einem Trifluoracetatpuffer, welcher auf pH-Werte von 2 und 3 eingestellt war, konnte keine Desorption des Polyelektrolyts nachgewiesen werden. Erst bei Verwendung einer Pufferlösung (HCl/KCl), welche einen pH von 1,0 aufwies, wurde eine messbare Desorption der PEI-Schicht beobachtet. Bei einem weiteren Absinken des pH-Wertes durch Zugabe von HCl zersetzten sich die Trägerpartikel. Es ist daher nicht auszuschließen, dass die Ablösung des PEI eine Folge einer oberflächlichen Zersetzung der Partikeloberfläche ist.

### Beispiel 4: Stabilität der PEI-Schicht gegenüber niedrigem pH in Abhängigkeit von der PSS-Vorbeschichtung

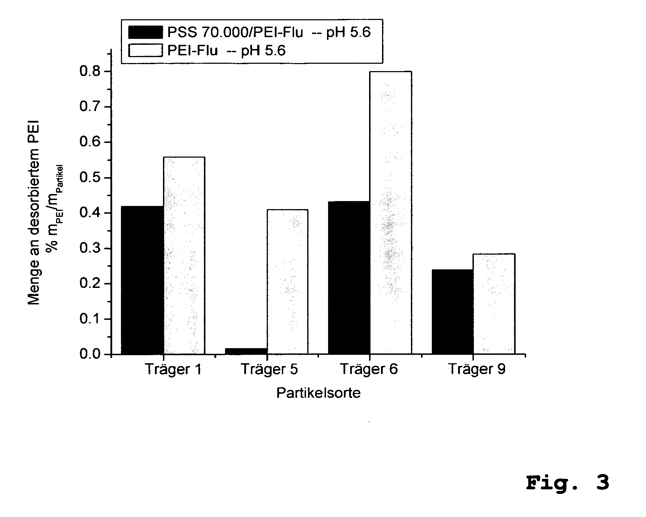

Jeweils 60 mg des zu untersuchenden Materials der Trägerkerne wurde in 1 ml Pufferlösung (pH 1,0; Fluka Chemika) suspendiert und 1 Stunde bei Raumtemperatur inkubiert. Die Mischung wurde zentrifugiert, der Überstand abpippetiert und mit 0,5 M Acetatpuffer pH 5,6 verdünnt. Die Konzentration des freigesetzten PEI-Flu wurde über die Extinktion der Probe ermittelt und daraus die prozentuale Menge an desorbiertem PEI pro Partikel berechnet. Die ermittelten Werte sind in Tabelle 9 zusammengefasst und in Fig. 3 graphisch wiedergegeben. Aus Fig. 3 geht hervor, dass eine PSS Vorbeschichtung bei allen Trägermaterialien eine Erhöhung der Stabilität der PEI-Schicht bewirkt. Dabei ist die Stabilität der Bindung des PEI an das Trägermaterial von der Art des Trägermaterials abhängig. Die höchste PEI-Adsorption wurde für das Trägermaterial 5 ermittelt.

**Tabelle 10: bei pH = 1 vom Trägerkern desorbierte Mengen an Polyethylenimin**

| Trägerkern | desorbiertes PEI (mit PSS Vorbeschichtung) | desorbiertes PEI (ohne PSS Vorbeschichtung) |
|---|---|---|
| Träger 1 | 0,41844 | 0,55825 |
| Träger 5 | 0,01582 | 0,40918 |
| Träger 6 | 0,43193 | 0,79868 |
| Träger 9 | 0,23186 | 0,28382 |

### Beispiel 5: Beschichtung von kationisch geladenen Trägermaterialien mit Polyethylenimin

Zur Herstellung der Beschichtungslösung wurden 0,25 g/l PSS-Rho in NaAc-Puffer 20 mM pH 5,6 und 0,2 M NaCl gelöst.

Als Träger für die Polyelektrolytbeschichtungen wurden Aluminiumoxide sowie Aluminiumoxidhydroxid ausgewählt, da diese bei einem pH Wert, bei dem die Beschichtung durchgeführt wird, einen PZC von ca. 9 aufweisen und positiv geladen sind. Daneben wurden auch noch synthetische Alumosilicate mit unterschiedlichem Aluminiumoxidanteil untersucht. Die untersuchten Materialien sind in Tabelle 11 zusammengefasst.

**Tabelle 11: untersuchte Materialien für Trägerkerne**

| Handelsname | Material |
|---|---|
| Pural^{®} SB | Boehmit |
| Pural^{®} SCC 150 | Boehmit |
| Pural^{®} TH 500 | Boehmit |
| Puralox^{®} KR-160 | Aluminiumoxid |
| Puralox^{®} SCC a | Aluminiumoxid |
| Siral^{®} 5 | Aluminiumoxid |

Alle angeführten Materialien wurden von Sasol Germany GmbH, A-ckermannplatz 1, 20537 Hamburg bezogen. Die physikalischen Daten sind in den Tabellen 12 sowie 13 und 14 aufgeführt.

**Tabelle 12. Chemische Zusammensetzung und charakteristische Daten von Siral^{®} 5**

| Zusammensetzung | Siral^{®} 5 |
|---|---|
| Al₂O₃-Gehalt (%) | 75 |
| LOI (%) | 25 |
| Al₂O₃ : SiO₂ (%) | 95:5 |
| C (%) | 0.2 |
| Fe₂O₃ (%) | 0.02 |
| Na₂O (%) | 0.005 |
| Eigenschaft | Wert |
| Schüttgewicht(g/l) | 450-650 |
| Mittlere Teilchengröße (d₅₀) [µm] | 50 |
| BET Oberfläche (m²/g) | 365 |
| Kumulatives Porenvolumen (BJH) für Porendurchmesser 1.7 - 300 nm (cm³/g) | 0,60 |
| Durchschnittlicher Poren-durchmesser (BJH) (nm) | 6,34 |
| Zetapotential Bei 0,1 Gew% in Acetat- Puffer pH 5,6 | |

**Tabelle 13: Übersicht über kationisch geladene Materialien für Trägerkerne (chemische Zusammensetzung)**

| Zusammensetzung | Pural^{®} SB | Pural^{®} TH 500 | Pural^{®} SCC 150 | Puralo^{®} SCCa-150/230 | Puralox^{®} KR-160 |
|---|---|---|---|---|---|
| Al₂O₃-Gehalt (%) | 74 | 82,8 | 74 | 98 | 95,1 |
| LOI (%) | n.d. | n.d. | n.d. | 2 | n.d. |
| Na₂O (%) | 0, 002 | n.d. | 0, 002 | 0, 002 | 0, 002 |

**Tabelle 14: physikalische Eigenschaften verschiedener kationisch geladener Trägerkerne**

| | | | | | |
|---|---|---|---|---|---|
| Eigenschaften | Pural^{®} SB | Pural^{®} TH 500 | Puralox^{®} KR-160 | Pural^{®} SCC 150 | Puralox^{®} SCCa-150/230 |
| Schüttge-wicht(g/l) | 650-850 | 300 | 270 | 700-850 | 600-850 |
| Mittlere Teil-chengröße (d₅₀) [µm] | 45 | 45 | 35 | 60-150 | 60-150 |
| Wassergehalt (%) | Zu best. | Zu best. | Zu best. | Zu best. | Zu best. |
| pH (10 Gew.-% in Wasser) in Wasser) | Zu best. | Zu best. | Zu best. | Zu best. | Zu best. |
| BET Oberfläche (m²/g) | 295 | 30 | 160 | 302 | 216 |
| Kumulatives Porenvolumen (BJH) für Po-rendurchmesserrendurchmesser 1.7 - 300 nm (cm³/g) | 0,43 | 0,12 | 0,80 | 0,41 | 0,51 |
| Durchschnitt-licher Poren-durchmesserdurchmesser (BJH) (nm) | 5,0 | 15,7 | 17,2 | 4,7 | 9,4 |
| Zetapotenti-al[mV] * Bei 0,1 Gew% in Acetat-Puffer pH 5,6 | +19,0 | +21,1 | +23,8 | +16,8 | +26,1 |

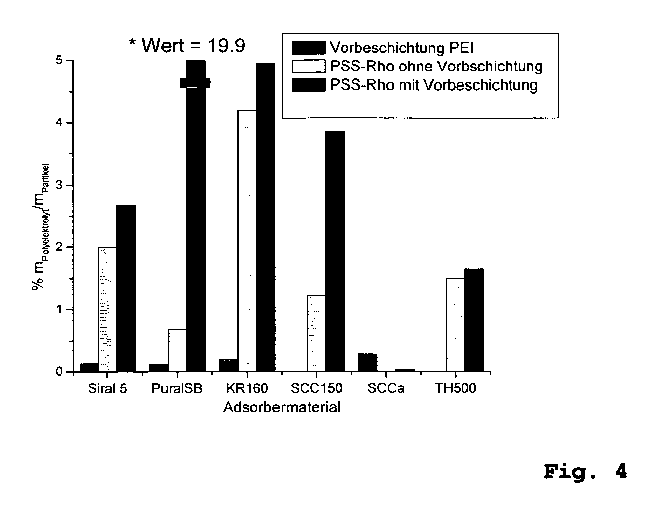

Die Trägermaterialien wurden wie in Beispiel 3 beschrieben mit PEI-FLU beschichtet und die Menge an adsorbiertem Material aus Messungen des im Überstand verbliebenen Polyelektrolyts bestimmt. Die Beladungen sind in Tabelle 15 zusammengefasst sowie graphisch in Fig. 4 wiedergegeben. Im nächsten Schritt wurden die Trägermaterialien mit der PSS-Rho-Beschichtungslösung beschichtet, wobei eine erste Versuchsreihe mit Trägermaterialien durchgeführt wurde, welche nicht vorbeschichtet waren. In einer zweiten Reihe wurde Trägermaterial verwendet, welches mit PEI-Flu vorbeschichtet worden war. Die Menge an adsorbiertem PSS-Rho wurde ebenfalls quantitativ bestimmt. Die adsorbierten Mengen sind ebenfalls in Tabelle 15 aufgeführt sowie in Fig. 4 wiedergegeben.

**Tabelle 15: auf kationisch geladenen Trägerkernen adsorbierte Mengen an Polyelektrolyt (% m_{Polyelektrolyt}/m_{Partikel})**

| Träger | adsorbiertes PEI | adsorbiertes PSS-Rho |
|---|---|---|
| Siral^{®} 5 | 0,13 | 2,68 |
| Pural^{®} SB | 0,12 | 19,9 |
| Puralox^{®} KR 160 | 0,19 | 4,95 |
| Pural^{®} SCC 150 | 0 | 3,85 |
| Puralox^{®} SCCa | 0,28 | 0,03 |
| Pural^{®} TH 500 | 0 | 1,65 |

Wie Fig. 4 zeigt, ist die Menge an adsorbiertem PEI relativ gering. Die Vorbeschichtung hat jedoch deutlichen Einfluss auf die nachfolgende Adsorption von PSS-Rho. Bei anderen verwendeten Trägermaterialien wurde nach einer Vorbeschichtung mit PEI eine höhere Menge an PSS-Rho adsorbiert. Besonders bei Pural^{®} SB wurde nach Vorbeschichtung mit PEI im Vergleich zum unbeschichtetem Material etwa die 10-fache Menge an PSS-Rho adsorbiert. Dies geht vermutlich auf die bessere Ausnutzung der Porosität zurück.

Bei den adsorbierten Massen muss berücksichtigt werden, dass das Molekülgewicht der Monomereinheit (1 Ladung) bei PSS 206 g/mol beträgt, wogegen es bei Polyethylen nur maximal 79 g/mol erreichen kann.

### Beispiel 6: Einfluss der Vorbeschichtung auf die Stabilität der Beschichtung von kationischen Trägermaterialien

Die in Beispiel 4 enthaltenen, ohne Vorbeschichtung mit PSS-Rho beschichteten Proben Siral 5 (2% PSS) und Puralox KR 160 (4,2 % PSS) wurden in Wasser, bzw. einer 1 M NaCl-Lösung sowie in Puffern, welche einen pH-Wert von von 7,9 bzw. 10,5 aufwiesen, suspendiert und 14 Stunden bei Raumtemperatur inkubiert. Nach Zentrifugieren wurde der Überstand abpippetiert und die im Überstand enthaltene desorbierte Menge an PSS-Rho durch UV/Vis-Spektroskopie bestimmt. In Tabelle 14 sind die Ergebnisse zusammengefasst.

**Tabelle 16: Desorbierte Mengen an PSS-Rho (prozentualer Wert bezogen auf das Partikelgewicht)**

| Lösung | Siral 5 | m_{Partikel} (mg) | Desorption | Pura-lox KR 160 | m_{Partikel} (mg) | Desorption |
|---|---|---|---|---|---|---|
| H₂O | 0 | 12,2 | 0% | 0,010 | 12,1mg | 0,05% |
| 1M NaCl | 0,008 | 12,2 | 0,04% | 0,019 | 12,1mg | 0,1% |
| 50mM TRIS-Puffer pH 7 | 0,013 | 12,2 | 0,07% | 0,023 | 12,1mg | 0,12% |
| 50 mM Ammoniak/ Ammoniumchlorid pH 9 | 0,056 | 12,2 | 0,29% | 0,050 | 12,1mg | 0,26% |
| 100 mM Ammoniak/ Ammoniumchlorid pH 10,5 | 0,179 | 12,2 | 0,95% | 0,185 | 12,1mg | 0,98% |

Die untersuchten Trägermaterialien zeigten eine hohe Stabilität gegenüber hoher Salzkonzentration. Unter stark basischen Bedingungen wird die Beschichtung aus PSS-Rho zunehmend abgelöst.

### Beispiel 7: Desorptionsmessungen

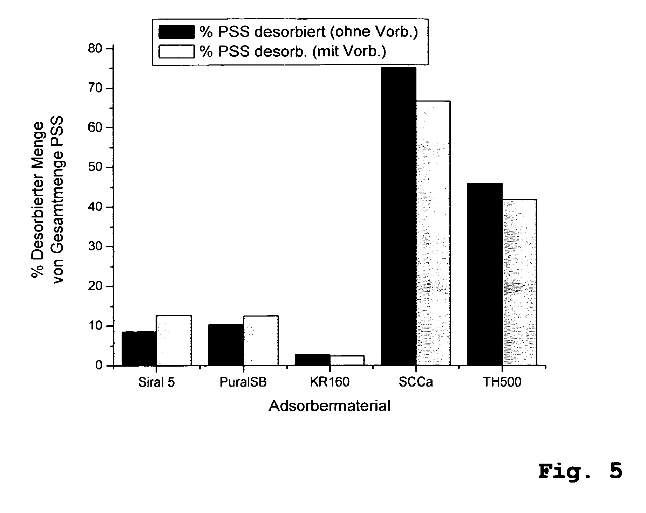

Die in Beispiel 4 erhaltenen, mit PSS-Rho beschichteten Trägermaterialien wurden jeweils in 50 mM NH₃/NH₄Cl-Puffer (pH 9,5) suspendiert und für 14 Stunden bei Raumtemperatur inkubiert. Die Proben wurden abzentrifugiert und die Menge an desorbiertem PSS-Rho im Überstand durch UV/Vis-Spektroskopie bestimmt. Die desorbierte Menge ist jeweils auf die Menge an adsorbiertem PSS bezogen. Die Ergebnisse sind in Tabelle 17 zusammengefasst und in Fig. 5 graphisch wiedergegeben.

**Tabelle 17: Prozentuale Menge an desorbiertem PSS bei den beschichteten Adsorbermaterialien unter Streßbedingungen**

| Trägermaterial | % PSS desorbiert (ohne Vorbeschichtung) | % PSS desorbiert (mit Vorbeschichtung) |
|---|---|---|
| Siral^{®} 5 | 8,5 | 12,6 |
| Siral^{®} 40 | 58,4 | 7,5 |
| Pural^{®} SB | 10,3 | 12,6 |
| Puralox^{®} KR160 | 2,8 | 2,4 |
| Puralox^{®} SCC a | 75 | 66,7 |
| Pural^{®} TH 500 | 46 | 41,8 |

Der Stabilisierungseffekt durch die Vorbeschichtung mit PEI ist zwar weniger deutlich ausgeprägt als bei den in Beispiel 3 untersuchten anionischen Adsorbermaterialien. Speziell bei Siral^{®} 40 konnte jedoch eine sehr starke Stabilisierung erreicht werden.

## Patentansprüche

1. Trägermaterial, zumindest umfassend:
a.) einen inerten Trägerkern, welcher eine Oberfläche mit einer positiven oder negativen Gesamtladung aufweist;
b.) eine auf dem inerten Trägerkern angeordnete und sich an diesen anschließende erste Schicht aus einem ersten Polyelektrolyt, welcher eine zur Oberflächenladung des inerten Trägerkerns gleichsinnige Gesamtladung aufweist;
c.) eine auf der ersten Schicht aus einem ersten Polyelektrolyt angeordnete zweite Schicht aus einem zweiten Polyelektrolyt, welcher eine zur Gesamtladung des ersten Polyelektrolyts gegensinnige Gesamtladung aufweist.

2. Trägermaterial nach Anspruch 1, wobei der inerte Trägerkern aus einem anorganischen Material aufgebaut ist.

3. Trägermaterial nach Anspruch 2, wobei das anorganische Material ausgewählt ist aus der Gruppe von Siliziumdioxid, Aluminiumoxid, Alumosilicat, Titanoxid, Zirkonoxid, Zeolithen, Tonen, insbesondere wärmebehandelten Tonmineralien.

4. Trägermaterial nach einem der vorhergehenden Ansprüche, wobei der erste Polyelektrolyt geladene Wiederholungseinheiten aufweist, welche eine kationische und/oder anionische Gruppe tragen und der Anteil der geladenen Wiederholungseinheiten größer als 20 mol-% gewählt ist, bezogen auf die Gesamtheit der Wiederholungseinheiten.

5. Trägermaterial nach einem der vorhergehenden Ansprüche, wobei der erste Polyelektrolyt ausgewählt ist aus kationischen stickstoffhaltigen Polymeren.

6. Trägermaterial nach Anspruch 5, wobei das kationische stickstoffhaltige Polymer ausgewählt ist aus der Gruppe von Polyaminen, Polyiminen, Polypyridinen, Polyimidazolen, sowie Chitosan.

7. Trägermaterial nach einem der vorhergehenden Ansprüche, wobei der erste Polyelektrolyt von einem anionischen Polymer gebildet wird, welches eine negative Gesamtladung aufweist.

8. Trägermaterial nach Anspruch 7, wobei das anionische Polymer ausgewählt ist aus der Gruppe von Polycarbonsäuren, Polysulfonsäuren, Polyboronsäuren, Polyphosphaten, Polyphosphonaten und Polysulfaten.

9. Trägermaterial nach einem der vorhergehenden Ansprüche, wobei der zweite Polyelektrolyt ein Molekülgewicht im Bereich von 5.000 bis 5.000.000 g/mol aufweist.

10. Trägermaterial nach einem der vorhergehenden Ansprüche, wobei der zweite Polyelektrolyt anionisches Polymer ist, welches eine negative Gesamtladung aufweist.

11. Trägermaterial nach einem der vorhergehenden Ansprüche, wobei auf der zweiten Schicht aus dem zweiten Polyelektrolyt zumindest eine weitere Schicht aus einem Polyelektrolyt angeordnet ist, wobei die Polyelektrolyten aufeinander angeordneter Schichten eine gegensinnige Gesamtladung mit gegensinnigen Vorzeichen aufweisen.

12. Verfahren zur Herstellung eines Trägematerials nach einem der Ansprüche 1 bis 11 mit zumindest den folgenden Schritten:
a.) Bereitstellen eines inerten Trägerkerns, welcher eine Oberfläche mit einer positiven oder negativen Gesamtladung aufweist;
b.) Bereitstellen eines ersten Polyelektrolyts, welcher eine zur Oberflächenladung des inerten Trägerkerns gleichsinnige Gesamtladung aufweist;
c.) Beschichten des inerten Trägerkerns mit dem ersten Polyelektrolyt;
d.) Bereitstellen eines zweiten Polyelektrolyts, welcher eine zum ersten Polyelektrolyt gegensinnige Gesamtladung aufweist;
e.) Beschichten des mit dem ersten Polyelektrolyt beschichteten inerten Trägerkerns mit dem zweiten Polyelektrolyt.

13. Verfahren nach Anspruch 12, wobei zumindest ein Biomolekül auf dem Trägermaterial gebunden wird.

14. Verwendung eines Trägermaterials nach einem der Ansprüche 1 bis 11 zur Immobilisierung von Biomolekülen.

15. Verwendung eines Trägermaterials nach einem der Ansprüche 1 bis 11 zur Auftrennung von Gemischen von Biomolekülen.
